# EUROPEAN PATENT SPECIFICATION

(11) **EP 2 249 862 B1**
(45) Date of publication and mention of the grant of the patent: **09.07.2014**
(21) Application number: 09700993.0
(22) Date of filing: 07.01.2009
(51) Int. Cl.: A61K 38/36, A61K 38/18, C07K 1/14, C07K 1/36

(54) **METHOD FOR PRODUCING A CLOTTABLE CONCENTRATE OF PLATELET GROWTH FACTORS**
VERFAHREN ZUR HERSTELLUNG EINES GERINNBAREN KONZENTRATES DER THROMBOZYTEN-WACHSTUMSFAKTOREN
ROCÉDÉ DE PRÉPARATION DE CONCENTRÉ COAGULABLE DE FACTEURS DE CROISSANCE DES PLAQUETTES

(30) Priority: 07.01.2008 EP 08290011
(43) Date of publication of application: 17.11.2010
(73) Proprietor: GwoWei Technology Co., Ltd., Taipei City 111 (TW)
(72) Inventor: BURNOUF, Thierry, F-59249 Aubers (FR); SU, Cheng-Yoa, Taipei (TW)
(74) Representative: Hirsch & Associés
(86) International application number: PCT/IB2009/000013
(87) International publication number: WO 2009/087560

(56) References cited:
- EP-A- 1 172 115
- DE-A1- 19 841 698
- US-A- 5 099 003
- US-A1- 2004 092 451
- DATABASE WPI Week 200676 Thomson Scientific, London, GB; AN 2006-728447 XP002474869 & CN 1 752 102 A (WUHAN HAITE BIOLOGICAL PHARM CO LTD) 29 March 2006 (2006-03-29)

## Description

### FIELD OF THE INVENTION

The present invention relates to the field of platelet derivatives and more specifically to the field of growth factors concentrates which are obtained from platelets. The present invention also relates to methods for preparing such growth factors concentrates.

### BACKGROUND

The mechanisms and pathways that govern tissue wound healing and tissue regeneration have been studied in great details, showing in particular that the cellular and molecular events resulting after a traumatic injury are mostly shared by the different tissues of the body. A common mechanism thus includes early and late inflammation phases, proliferation and migration of cell, angiogenesis, granulation tissue formation and finally matrix formation and remodelling.

Interestingly, this cascade of events is initiated immediately after injury by the formation of a blood clot characterized by cross-linked fibrin and by a variety of proteins such as vitronectin, fibronectin and thrombospondin, which prevents further bleeding, and serves as a matrix for invading cells while providing at the same time a barrier against invading pathogens. In addition, this initial clot acts as a reservoir of cell derivatives which are required during the later stages of the healing process.

It is in particular assumed that all the phases of tissue repair process are mediated and controlled by a wide range of factors, cytokines and proteins that modulate cell function through direct physical interactions with extracellular domain of transmembrane receptors. The latter transducer secondary signals, thereby controlling diverse aspects of subcellular biology. Although the role of all the components involved in tissue regeneration is only partially elucidated, the potential benefits of many of them have been demonstrated, especially for platelet derivatives, and more particularly for platelet-derived growth factors. Growth factors derived from platelets are particularly known to exhibit chemotactic and mitogenic properties and appear to be directly involved in soft and hard tissue healing and regeneration such as chemotaxis, cell proliferation, angiogenesis, extracellular matrix deposition and remodelling and in the enhancement of cell proliferation. Further, platelet derivatives are also indirectly involved in closely related biological functions such as the stimulation of chemokines and cytokines production by bystander cells such as fibroblasts, macrophages, endothelial cells, or lymphocytes.

Preparations enriched in platelets, as well as platelets gels, glues and releasates are therefore increasingly used alone or in combination with grafting biomaterials, as a source of platelet derivatives or platelet growth factors for various clinical or therapeutic applications.

Such platelet-containing or -derived preparations, have been found to be particularly beneficial for treating large bone defects, complex cranioplasties, and chronic wounds, for example in oral and maxillofacial, orthopaedic, periodontic and plastic surgery, as well as in the treatment of chronic ulcers, bone and soft tissue regeneration in dentistry and oral implantology, and in the treatment of musculoskeletal conditions.

Furthermore, platelets releasates were shown to be of particular interest for *ex vivo* expansion and differentiation of mesenchymal stem cells, as well as for accelerating the proliferation of chondrocytes, endothelial cells and fibroblasts, thereby supporting prospects of expanding therapeutic benefits for cartilage regeneration and wound healing.

Topical platelet-derived preparations, such as gels, glues and releasates are usually prepared by mixing platelet concentrates with an activator capable of inducing the release of the platelet granules content and in mimicking the physiological activation of platelets. The activation step is generally performed by direct addition of exogenous thrombin or through the use of calcium chloride (CaCl₂), which counterbalance the effects of the anticoagulant added during the collection of blood or platelets, and trigger the coagulation cascade and the release of endogenous thrombin. The endogenous and exogenous thrombin also induce polymerisation of fibrinogen and formation of a fibrin-based biomaterial (a clot), thereby leading to the subsequent release of a multifaceted blend of components entrapped in platelets, comprising for example various platelet growth factors.

More recently, new approaches were developed, consisting in the production of recombinant platelet growth factors in conventional expression systems, such as Regranex (human PDGF-bb) (Janssen Cilag Internat.). However, the number of available recombinant growth factors remains extremely limited, which is in part due to the difficulty to isolate, identify, clone and express these growth factors. Furthermore, platelet-derived preparation still provide a supplementary benefit over the use of single recombinant factors, since a synergistic effect of growth factors combination can be observed

One of the main drawbacks of the existing platelet-containing or platelet-derived preparations belongs to the lack of a suitable standardization and definition of such preparations, which has led to variability in the characteristics of platelet-rich products with variable therapeutic effects. Indeed, the simple assumption that platelet concentration predicts the level of growth factors in platelet derivatives is not clear, since the technical characteristics of the processes used to collect and/or to activate platelets impact the content in growth factors and the resulting clinical effects. The heterogeneity of the resulting products might for instance depend on parameters as different as platelets concentration, presence of white-blood cells in the starting material, kind, age, and storage conditions of the starting material and means of activation. It is thus quite obvious that these variables lead to important differences among products, and, in turn, to distinct biological properties and clinical efficacy such as healing capacity.

For instance, when solutions/gels containing intact platelets are loaded directly on the wound site, the activation of platelets is associated with a quick formation of a fibrin clot, but, as well, in an incomplete lysis of platelets. Thus, a variable amount of intact platelets remains entrapped inside the fibrin clot, thereby rendering it difficult to determine the real amount of growth factors released.

Further, when platelets gels and/or growth factors preparations are obtained by a thrombin activation process resulting in platelets activation, these platelets derivatives are fully depleted in fibrinogen and are no longer clottable, thus requiring to be mixed with exogenous natural or synthetics products before to be applied on the wound site.

Further, most therapeutic topical platelet derivatives are prepared from autologous sources. This means that autologous platelet concentrates are prepared for point-of-care applications from blood donated by the patient a few hours or a few days prior to surgery. Their use is thus largely reserved for planned surgeries requiring a limited volume of platelet gel and for patients healthy enough to donate blood. In addition, the manufacture of autologous preparations at the surgical site has the disadvantage of being often carried out under poorly controlled conditions, therefore lacking the standardization needed to ensure reproducible growth factors release and clinical efficacy.

There is therefore also a strong need for a method allowing the anticipated preparation of platelet derivatives and more specifically of platelet growth factors concentrates under standardized conditions, thereby rendering it possible to formulate a tailored preparation for each specific physiopathological situation.

Furthermore, another major drawback of the existing platelet-derived products belongs to the inevitable presence of intact blood cells or of important blood cells fragments, thereby triggering the development of an immune response with antibodies directed toward donor antigens when platelet-derived products are from heterologous source. Immune responses to allogeneic antigens may result in severe consequences, such as those observed with transfusion reactions, and include, for instance alloimmunization and hemolytic complications in patients.

Furthermore, another major drawback of the existing platelet-derived products belongs to the inevitable infectious risks associated to the use of biological products. Indeed, in spite of the high level of safety of single-donor human allogeneic platelet concentrates which are tested by modem technologies in countries with highly developed regulatory environment and blood collection services, and although standard blood bank methods such as apheresis or platelet preparation from whole blood guarantee sterile preparation conditions as far as possible, viral and bacterial transmission is still susceptible to occur with the use of platelet derivatives. Viral innocuity of platelet derivatives and of platelet growth factors is thus highly desirable to ensure optimal viral safety margin of allogeneic platelet preparations for topical use.

Finally, the aging population and the increasing number of chronic-diseases pose a major and growing health problem which will have to be managed in a near future. Although a wide variety of therapeutic treatments already exist, including for example surgery, antibiotics administration, nutritional supplementation, or tissue grafts, there is an increasing need for highly efficient and economically attractive components to be used in tissue wound healing and in tissue regeneration.

The inventors have now found surprisingly and after intensive research, that these objectives can be reached with the hereinafter described clottable concentrate of platelets growth factors and with the preparation method thereof.

The method of the invention allows in particular the easy, rapid and efficient preparation of platelet derivatives and more particularly of clottable concentrate of platelets growth factors from a starting platelet concentrate or a pool of platelet concentrates. In particular, the method of the invention increases significantly the recovery of all, at least of the more important platelet growth factors, and more particularly of growth factors PDGF, TGF-ß and EGF, when compared to other methods disclosed in the state of the art.

Further, the method of the invention allows the dissolution of lipid membranes, therefore inactivating lipid-enveloped viruses, and other pathogens like, for instance, bacteria and parasites such as protozoae, as well as the removal of plasma and platelet lipids which are present in the starting platelet concentrate.

The method of the present invention thus renders it possible to provide virally- inactivated clottable platelet growth factors concentrates which could be efficiently standardized for use in therapeutic treatments or cell therapy.

Considering the fact that, in part due to the risk of bacterial contamination, platelets have a limited shelf life of 5 or 7 days, when clinically used intravenously for the correction of quantitative or functional thrombocytopenia, a high number of platelet units older than 5 or 7 days are usually discarded each year. In allowing expired platelets stocks to be used for the preparation of platelet-derived concentrates, the method of the invention finally reveals an extremely promising economical interest.

### SUMMARY OF THE INVENTION

THE PRESENT INVENTION IS DEFINED BY THE CLAIMS. The present application describes a clottable concentrate virally inactivated of platelet growth factors suitable for therapeutic and/or cosmetic use, obtainable from a non-activated pool of platelet concentrates, wherein non-encapsulated and encapsulated viruses have been inactivated, and comprising both fibrinogen and factor XIII, said concentrate being free of intact platelets.

Preferably this clottable virally inactivated concentrate of platelet growth factors comprises the growth factors PDGF, TGF-β, IGF, EGF, CTGF, bFGF and VEGF.

Preferably said clottable virally inactivated concentrate of platelet growth factors does not induce blood cell-related transfusion reactions.

Preferably said clottable virally inactivated concentrate of platelet growth factors comprises at least one protein selected in the group consisting of fibronectin, vitronectin, thrombospondin, von Willebrand factor and coagulation factors II, V, VII, VIII, IX, X, and XI.

An object of the present invention is a method for preparing a clottable virally inactivated concentrate of platelet growth factors containing both fibrinogen and factor XIII, comprising the following steps :
a) contacting a non-activated starting platelet concentrate with a solvent and/or a detergent, wherein said solvent is preferably selected in the group consisting of di- or trialkylphosphates, di or trialkylphosphates with different alkyl chains, and is preferably the tri-n-butylphosphate (TnBP), wherein said detergent is preferably selected in the group consisting of polyoxyethylene derivatives of fatty acids, partial esters of sorbitol anhydrides, non-ionic detergents, sodium deoxycholate and sulfobetaines, and is more preferably Triton X-45, Triton X-100 or Tween 80, and wherein the final concentration of each of said solvent and/or of said detergent preferably ranges from 0.2 to 5 % in volume with respect to the volume of the starting platelets concentrate;
b) incubating said starting platelet concentrate with the solvent and/or detergent for a period of at least 5 minutes to 6 hours, at a pH maintained in a range from about 6.0 to about 9.0, and at a temperature within the range of from 2°C to 50°C, and
c) removing the solvent and/or the detergent by oil extraction and/or chromatographic means, wherein oil extraction is preferably performed with a pharmaceutical grade oil, the oil being used in an amount of from 2 to 20 weight%, or from 5 to 15 weight % or from 5 to 10 weight %, based on the weight of the mixture of the platelet concentrate with the solvent and/or the detergent, and wherein the chromatographic means preferably comprise an hydrophobic (reversed phase) column, preferably a C18 silica packing material or a SDR (Solvent-Detergent removal) hyper D, or an adsorption chromatographic column such as an anionic and/or cationic chromatographic column; said method comprising a further step of inactivating non-enveloped viruses, wherein this step is performed by nanofiltration, preferably by using a 10 to 75-nm pore size pilter membrane.

An object of the present invention is a method for preparing a clottable virally inactivated concentrate of platelet growth factors according to the claims and comprising the steps of contacting a platelet concentrate with a solvent and/or a detergent, incubating said platelet concentrate with the solvent and/or detergent for a period of at least 5 minutes to 6 hours, at a pH maintained in a range from about 6.0 to about 9.0, and at a temperature within the range of from 2°C to 50°C, preferably within the range of from 25°C to 45°C, and removing the solvent and/or the detergent by oil extraction and/or chromatographic means.

In a preferred embodiment, the solvent used in the method of the invention is selected in the group consisting of di- or trialkylphosphates, di or trialkylphosphates with different alkyl chains, and is preferably the tri-n-butylphosphate (TnBP).

In a preferred embodiment, the detergent used in the method of the invention is selected in the group consisting of polyoxyethylene derivatives of fatty acids, partial esters of sorbitol anhydrides, non-ionic detergents, sodium deoxycholate and sulfobetaines, and more preferably in the group consisting in Triton X-45, Triton X-100, Tween 80 and Tween 20.

In a preferred embodiment, the final concentration of each of said solvent and/or of said detergent used in the method of the invention ranges from 0.2 to 5 %, preferably from 0.2 to 2 % in volume with respect to the volume of the starting platelets concentrate. In a preferred embodiment of the method of the invention, the starting platelet concentrate is contacted either with 2% TnBP only, or with 1% TnBP and 1% Triton X-45, based on the volume of the platelet concentrate.

Further, in a preferred embodiment of the present invention, oil extraction is performed with a pharmaceutical grade oil, the oil being used in an amount of from 2 to 20 weight %, or from 5 to 15 weight % or from 5 to 10 weight %, based on the weight of the mixture of the platelet concentrate with the solvent and/or the detergent.

In another preferred embodiment, chromatographic means, such as those comprising C18 silica packing material, or SDR (Solvent-Detergent removal) hyper D, are used to remove the solvent and/or the detergent.

In one instance of the present disclosure, the nanofiltration similar to a 10 to 75-nm pore size filter membrane, is done by using viral removal membranes.

In another preferred embodiment, the method of the invention further comprises an ultrafiltration step designed to concentrate the growth factors and the clottable fibrinogen to the levels optimally adapted to therapeutic applications or cell therapy, using ultrafiltration membranes of a cut value of 5000 Daltons or less.

In a preferred embodiment, the method of the invention comprises a preliminary step consisting in preparing a starting platelet concentrate, said starting platelet concentrate being prepared by apheresis or by buffy-coat isolation from whole blood, and being either fresh, expired and stored liquid or expired and stored frozen.

The present application also describes a method for forming a clot consisting in mixing the clottable virally inactivated concentrate of platelet growth factors of the invention or obtained by the method of the invention with thrombin. Preferably, the thrombin used in said method for forming a clot is of human origin. In a particular embodiment, 0.1 to I volume of thrombin, the activity of which ranges from 20 IU/ml to 1000 IU/ml, is mixed with I volume of the clottable virally inactivated concentrate of platelet growth factors of the invention or obtained by the method of the invention.

In another preferred embodiment, chromatographic means, such as those of the hydrophobic type e.g. comprising C18 silica packing material, or SDR (Solvent-Detergent removal) hyper D, are used to remove the solvent and/or the detergent; SD and/or DEAE type chromatography (anionic/cationic chromatography) can also be used to remove the solvent and/or the detergent.

The present application describes a pharmaceutical product or a scaffold comprising the clottable virally inactivated concentrate of platelet growth factors obtained by the method of the invention.

Another object of the present disclosure is a cosmetic use of the clottable virally inactivated concentrate of platelet growth factors of the invention or obtained by the method of the invention.

Another object of the present disclosure is a clottable virally inactivated concentrate of platelet growth factors of the invention or obtained by the method of the invention for use in regenerating bone, or for healing soft and hard tissue.

The present application also describes the use of the clottable virally inactivated concentrate of platelet growth factors obtained by the method of the invention to form a clot, or for bone regeneration or wound healing, or for *in vitro* or *ex vivo* cell culture.

### FIGURES

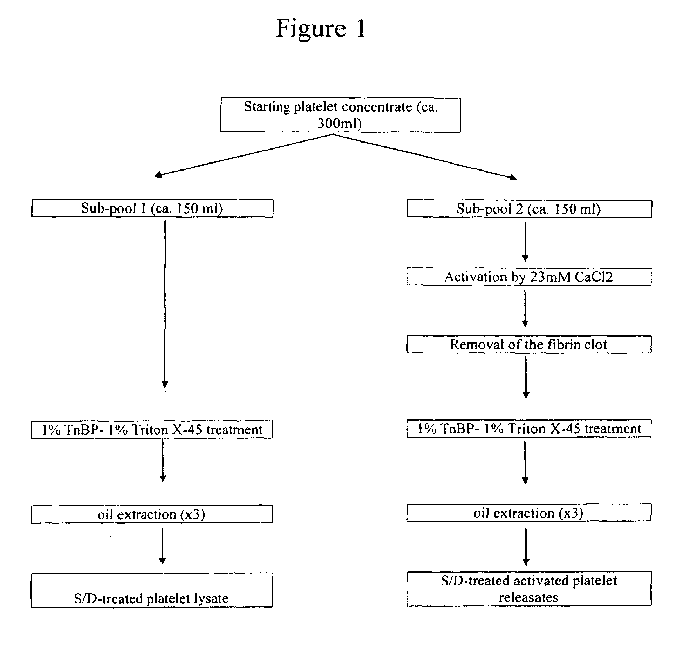

**Figure 1** **:** Study design: Apheresis platelet donations (N=8) were divided into two sub-pools. Sub-pool 1 was directly treated by 1% TnBP-1% Triton X-45 for 1h without prior CaCl₂ activation. Sub-pool 2 was first activated by 23 mM CaCl₂ and beads to form a platelet gel. After 1 h incubation, the clot was removed and the releasates treated by 1% TnBP-1% Triton X-45 for 1h. Both (solvent-detergent) S/D-treated solutions were extracted by oil (3 times) to remove the solvent and detergent. The content of growth factors was determined on the starting platelets, after activation, and after the S/D treatment-oil extractions.

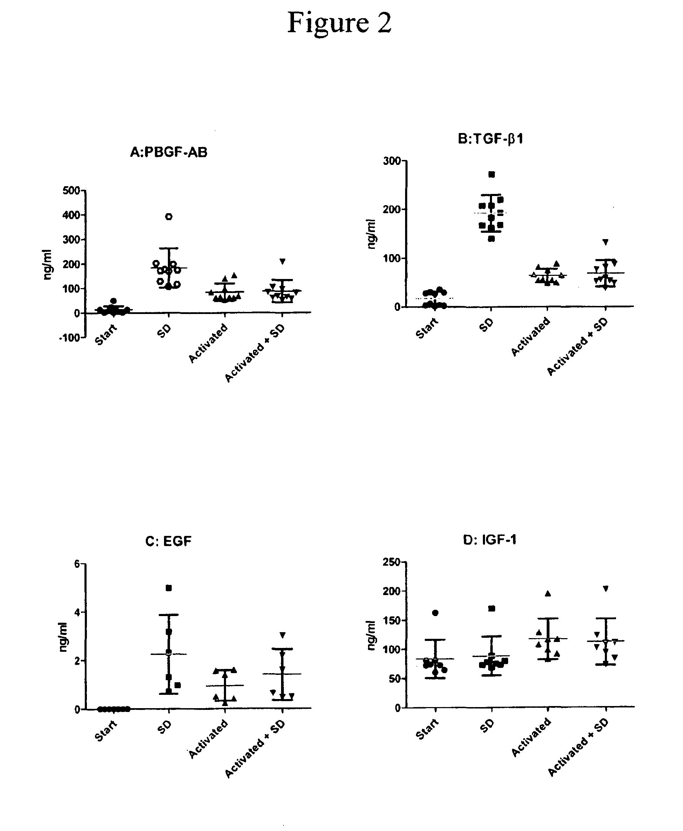

**Figure 2****:** Content (ng/ml) in (A) PDGF-AB; (B) TGF-ß1; (C) EGF; and (D) IGF in the starting platelet concentrate (start), after direct S/D treatment (1% TnBP-1% Triton X-45) (S/D), after CaCl2 activation of the starting platelet concentrate (Activated) and S/D treatment (1% TnBP- 1% Triton X-45) of the CaCl₂ activated platelet releasates (Activated + S/D). The S/D treatment was performed as described in the Material and Methods and included 3 oil extractions to remove the S/D agents.

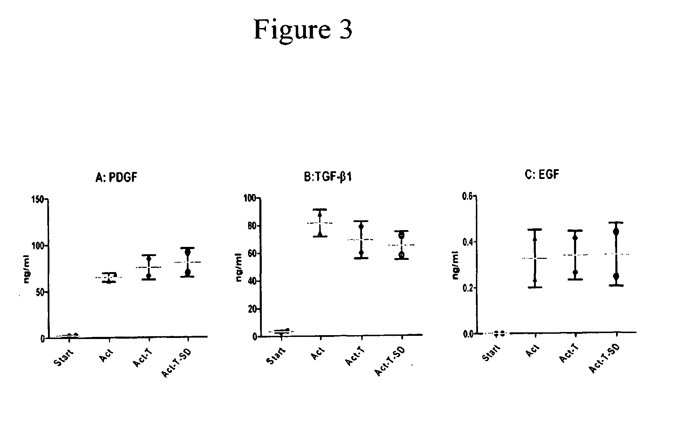

**Figure 3****:** Content (ng/ml) in (A) PDGF-AB; (B) TGF-ß1; and (C) EGF in the starting platelet concentrate (Start), after CaCl₂ activation of the starting platelet concentrate (Act) followed by bovine thrombin activation (Act-T) and S/D treatment (1% TnBP-1% Triton X- 45) (Act T-S/D). The S/D treatment was performed as described in the Material and Methods and included 3 oil extractions to remove the S/D agents.

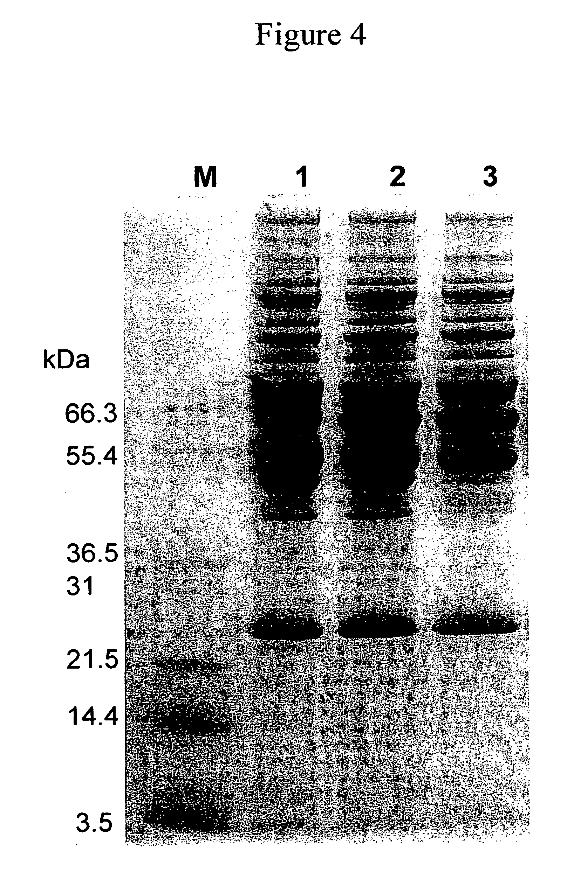

**Figure 4****:** Comparison of protein composition between the starting platelet concentrate (lane 1), the same platelet concentrate treated by the solvent-detergent process according to the invention (lane 2) and the same platelet concentrate after activation by CaCl₂ (lane 3). Samples were separated by SDS-PAGE on a 4%-12% gel, and proteins were colored by coomassie staining. Lane M corresponds to the protein marker (sizes in kiloDaltons are indicated on the left side of the gel).

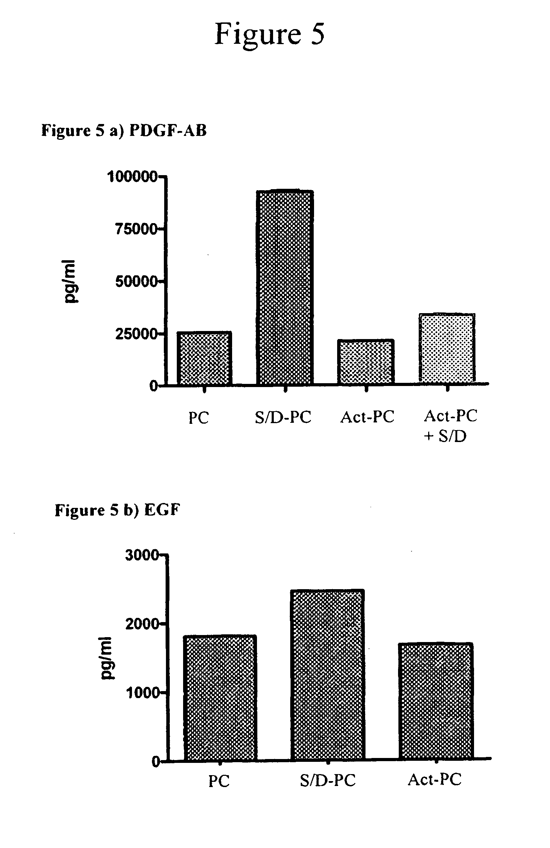

**Figure 5****:** Compared composition in PDGF-AB (Fig. 5a) and EGF (Fig. 5b) of concentrates respectively obtained from expired frozen platelets subjected to: S/D treatment (1% TnBP-1% Triton X-45) (S/D-PC), to CaCl₂ activation (Act-PC) or, optionally, to CaCl₂ activation followed by S/D treatment (Act-PC + S/D)

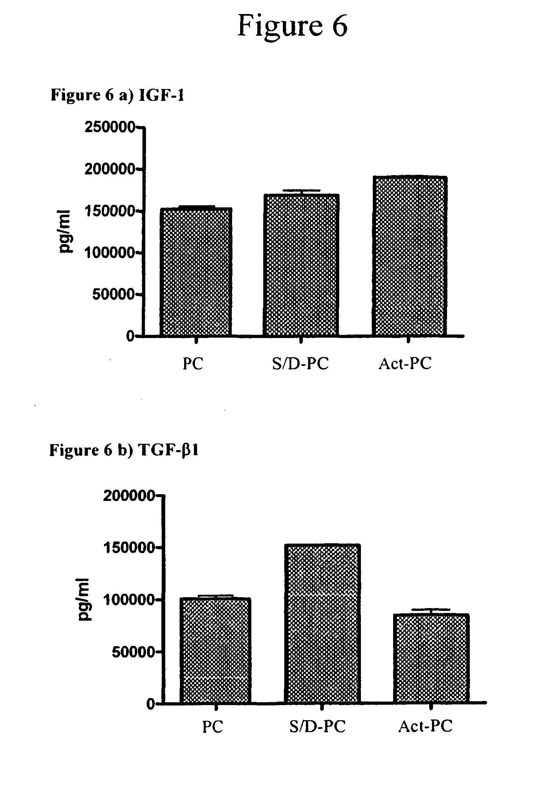

**Figure 6****:** Compared composition in IGF-1 (Fig. 6a) and TGF-β1 (Fig. 6b) of a concentrate respectively obtained from expired frozen platelets subjected to: S/D treatment (1% TnBP-1% Triton X-45) (S/D-PC) or to CaCl₂ activation (Act-PC).

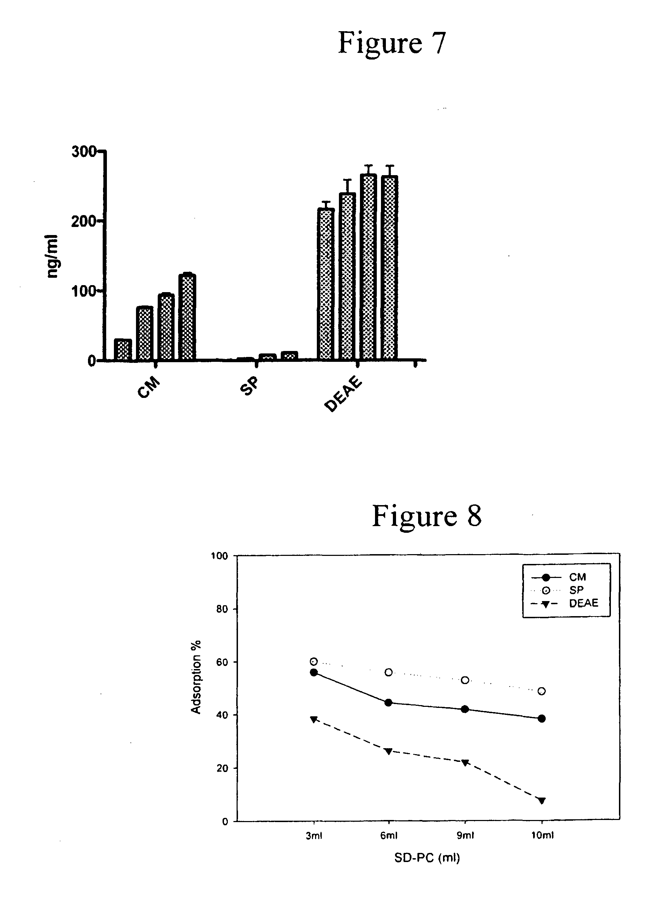

**Figure 7****:** Residual PDGF-AB content (in ng/mL) in the supernatants after adsorption tests on various chromatographic gels (CM, SP and DEAE). For each gel, the displayed results respectively correspond, from left to right, to a ratio of 3mL, 6mL, 9mL or 10 mL of SD-PC per 1 mL of gel.
**Figure 8****:** Adsorption % of VEGF on various chromatographic gels (CM, SP and DEAE).

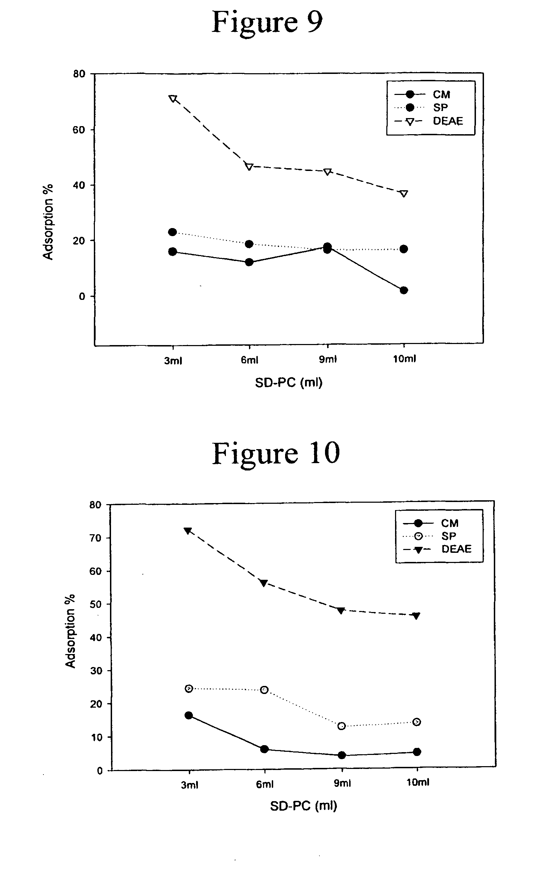

**Figure 9****:** Adsorption % of TGF-β1 on various chromatographic gels (CM, SP and DEAE).
**Figure 10****:** Adsorption % of EGF on various chromatographic gels (CM, SP and DEAE).

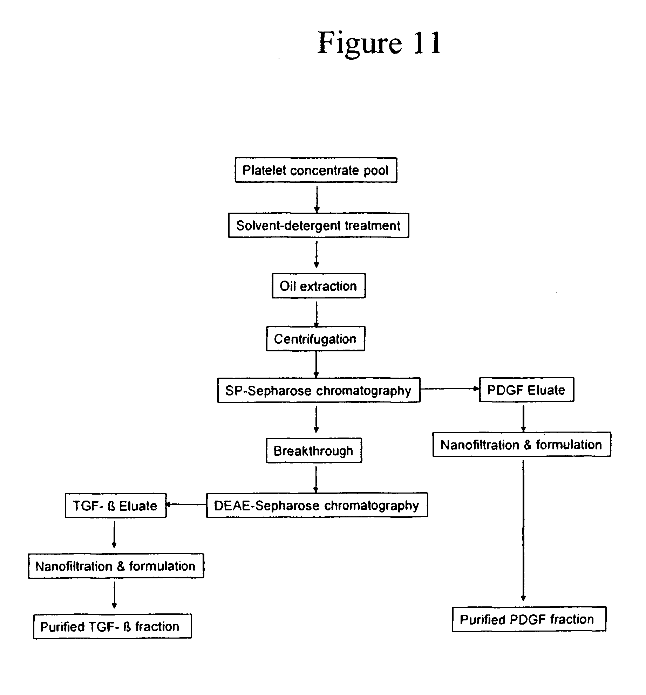

**Figure 11****:** Schematic representation of a particular embodiment of the method of the invention comprising I oil extraction followed by a first separation/purification step on SP-Sepharose. The PDGF eluate resulting from the SP-Sepharose both contains PDGF and VEGF Growth factors. The breakthrough fraction resulting from the SP-Sepharose is further separated/purified on DEAE-Sepharose, thus allowing to prepare a TGF-β eluate containing both TGF-β and EGF growth factors.

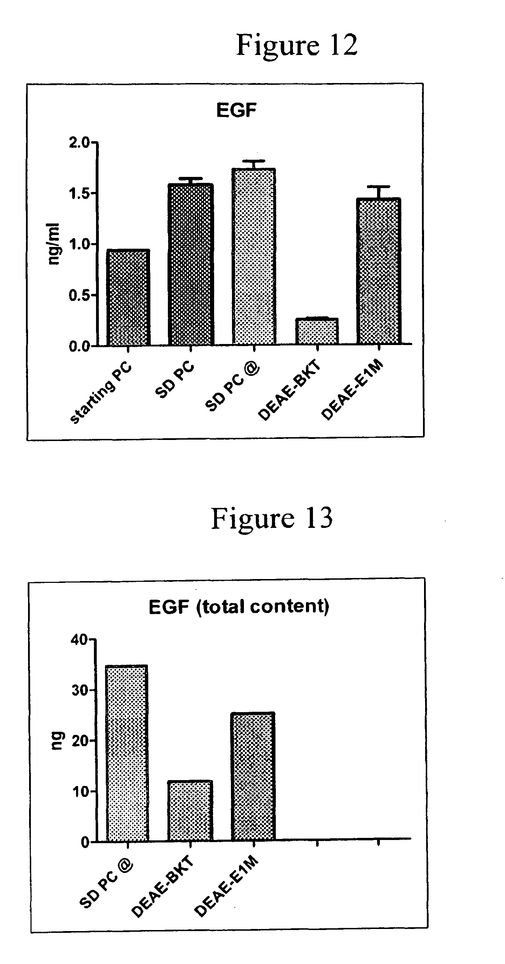

**Figure 12****:** EGF content expressed in ng/ml, in the starting PC, the PC treated by Solvent-Detergent, the PC treated by Solvent-Detergent after I oil extraction and centrifugation (SD-PC@), the breakthrough fraction after loading on DEAE Sepharose FF (DEAE-BKT) and the eluate recovered from DEAE Sepharose FF (DEAE-E1M).
**Figure 13****:** Total EGF content expressed in ng, in the PC treated by Solvent-Detergent after I oil extraction and centrifugation (SD-PC@), the breakthrough fraction after loading on DEAE Sepharose FF (DEAE-BKT) and the eluate recovered from DEAE Sepharose FF (DEAE-E1M).

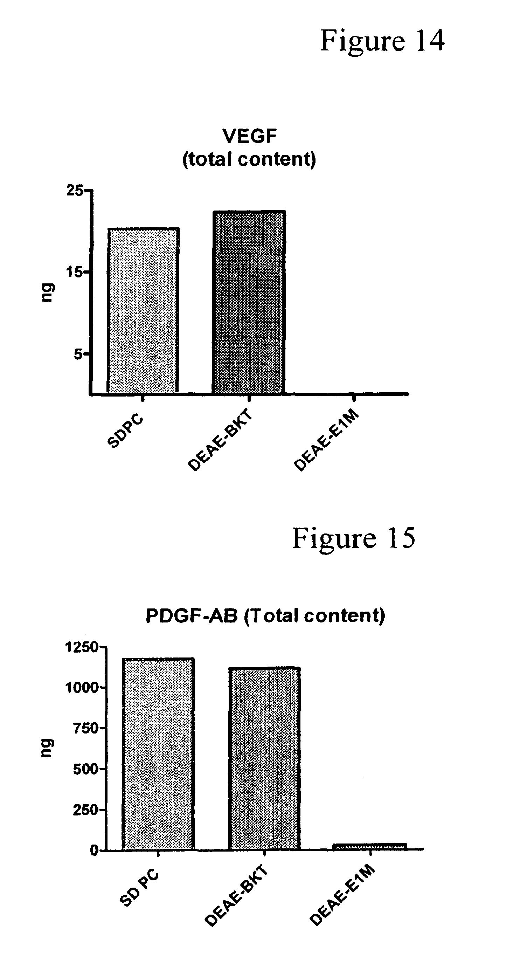

**Figure 14****:** Total VEGF content expressed in ng, in the PC treated by Solvent-Detergent after 1 oil extraction and centrifugation (SD-PC@), the breakthrough fraction after loading on DEAE Sepharose FF (DEAE-BKT) and the eluate recovered from DEAE Sepharose FF (DEAE-E1M).
**Figure 15****:** Total PDGF-AB content expressed in ng, in the PC treated by Solvent-Detergent after 1 oil extraction and centrifugation (SD-PC@), the breakthrough fraction after loading on DEAE Sepharose FF (DEAE-BKT) and the eluate recovered from DEAE Sepharose FF (DEAE-E1M).

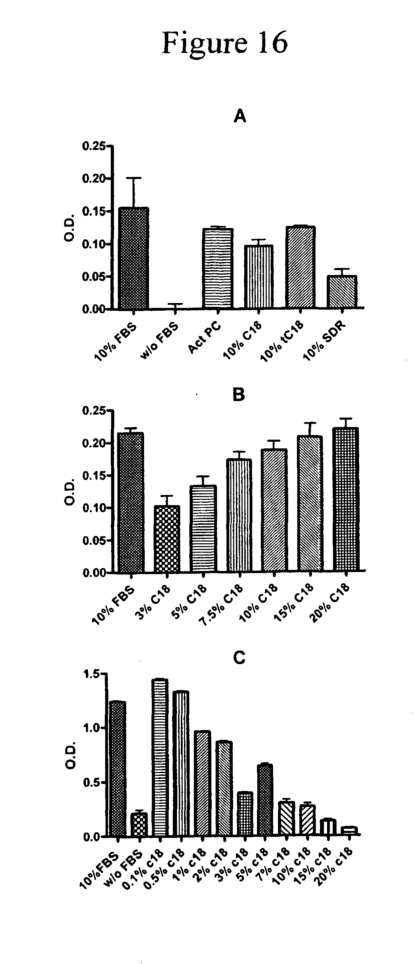

**Figure 16****:** Results of MTS assay. HEK293A fibroblast cells cultured in (A) D-MEM supplemented with 10% (v/v) FBS (10% FBS), without FBS (w/o FBS), 10% (v/v) activated PC releasate (Act PC), and 10% (v/v) of the HPGF mixture after chromatography on C18 (10%C18), tC18 (10%tC18), or SDR (10%SDR); in (B) D-MEM supplemented with 10% (v/v) FBS, or with 3, 5, 7.5, 10, 15, or 20% (v/v) of the HPGF mixture after C18. (C) SIRC fibroblast cells cultured in D-MEM supplemented with 10 % FBS, without FBS (w/o FBS), or with 0.1, 0.5, 1, 2, 3, 5, 7, 10, 15, or 20% (v/v) of the HPGF mixture after C18. Cell viability was determined after 5 days using the MTS cell proliferation assay following manufacturer's instructions.

### DETAILED SPECIFICATION

The present application describes a clottable virally inactivated concentrate of platelet growth factors for therapeutic and/or cosmetic use, obtainable from a non-activated pool of platelet concentrates, wherein non-encapsulated and encapsulated viruses have been inactivated, and comprising both fibrinogen and factor XIII, said concentrate being free of intact platelets.

As used in the present invention, by the term "clottable", it is meant that the concentrate of platelet growth factors obtained by the method of the invention comprises both fibrinogen and coagulation factor XIII, and is thereby capable to generate a clot upon mixing with a suitable activating agent, such as thrombin, when it is needed for therapeutic application.

Preferably, the concentration of fibrinogen in the clottable concentrate of platelet growth factors is preferably higher than 1, preferably higher than 1.5, preferably higher than 2.5 g/L of the concentrate, and the concentration of factor XIII is preferably higher than 0.5, preferably higher than 0.75, preferably higher than 1.0 iu/ml of the concentrate.

As used herein, the expression "cosmetic use" means a treatment intended to be contacted with the various superficial parts of the human body, in particular the skin, the hairs, the nails, the lips, the external genital organs, or with teeth and oral mucous membranes, so as to clean, perfume, protect, modify the aspect thereof or to maintain thereof in good condition.

As used in the present invention, by the expression "for therapeutic use", it is meant a curative or prophylactic treatment for treating conditions in human and/or animals, for restoring, correcting or modifying the physiological functions thereof through a pharmacological, immunological or metabolic effect.

The clottable virally inactivated concentrate of platelet growth factors obtained by the method of the invention is especially suitable for treating conditions in human and/or animals an/or for contacting superficial parts of the body thereof since it is both cleared of solvent and/or detergent, and virally safe.

By the expression "cleared of solvent and/or detergent", it is meant that the level of solvent and/or detergent in the clottable virally inactivated concentrate of platelet growth factors is extremely low, and preferably undetectable. It is indeed well-known from the skilled person in the art that elevated concentrations in solvent and/or detergent are directly linked with long-term toxicity, and more particularly with the onset of neurological conditions (such as disclosed in J.P.R. Pelletier, S. Transue, E.L. Snyder. Pathogen inactivation techniques. Best Practice & Research Clinical Haematology Vol. 19, No. 1, pp. 205-242, 2006).

Therefore, by the expression "cleared of solvent", it is meant in the present invention that the solvent level is of less than 100 ppm, preferably less than 50 ppm, preferably less than 20 ppm, more preferably less than 10 ppm, less than 5 ppm, and even more preferably less than 1 ppm.

Further, by the expression "cleared of detergent", it is meant in the present invention that the detergent level is of less than 500 ppm, preferably less than 250 ppm, more preferably less than 100 ppm, more preferably less than 50 ppm, and even more preferably less than 10 ppm.

By the expression "virally safe", it is meant that the clottable concentrate of platelet growth factors is substantially free of infectious virus, and more particularly of lipid-containing virus, such as for instance the human immunodeficiency virus (HIV), the hepatitis B virus (HBV), the hepatitis C virus (HCV, the West Nile virus (WNV), the TT virus, the Dengue virus, the cytomegalovirus (CMV), the Epstein Barr virus (EBV), the Human Herpes virus-8 (HHV-8), the simian foamy virus, the Severe Acute Respiratory Syndrome virus (SARS coronavirus) as well as other lipid-enveloped hepatitis viruses, cytomegaloviruses, lactic dehydrogenase viruses, herpes group viruses, rhabdoviruses, leukoviruses, myxoviruses, alphaviruses, arboviruses, paramyxoviruses, arenaviruses and coronaviruses. By "substantially free", it is meant that the clottable concentrate of platelet growth factors has an extent of inactivation of virus at least greater than 4log₁₀ as required to characterize robust viral reduction steps, by the Committee for proprietary medicinal products (CPMP) of the European Agency for the Evaluation of Medicinal Products (EMEA) in its "Note for guidance on virus validation studies" (Reference CPMP/BWP/268/95), and preferably greater than 5log₁₀ or even 6log₁₀, and is therefore unlikely to be responsible for transmission to patients of blood-borne infection due to lipid enveloped viruses.

The clottable virally inactivated concentrate of platelet growth factors obtained by the method of the invention further comprises the following functional growth factors : the platelet-derived growth factor (PDGF), either as heterodimers of A and B chains and/or as homodimers of A-A and /or B-B chains (PDGF-A, PDGF-AB, PDGF-B), the transforming growth factor (TGF-β) superfamily, comprising, among others, TGF-β1 and/or TGF-β2 and/or bone morphogenetic proteins (BMPs), the insulin-like growth factor (IGF), the epidermal growth factor (EGF), the connective tissue growth factor (CTGF), the basic fibroblast growth factor (bFGF), and the vascular endothelial growth factor (VEGF).

In a particular instance, the concentration of PDGF in the clottable virally inactivated concentrate of platelet growth factors is higher than 90, preferably higher than 100, preferably higher than 120, preferably higher than 150, preferably higher than 180 and preferably higher than 250 ng/ml of the concentrate.

In a particular instance the concentration of TGF-β1 in the clottable virally inactivated concentrate of platelet growth factors is higher than 100, preferably higher than 140, preferably higher than 160, preferably higher than 180, preferably higher than 250 ng/ml of the concentrate.

In a particular instance, the concentration of IGF in the clottable virally inactivated concentrate of platelet growth factors is at least similar to that of the starting platelet concentrate, and preferably higher than 65, preferably higher than 75, preferably higher than 80, preferably higher than 100 ng/ml of the concentrate.

In a particular instance, the concentration of EGF in the clottable virally inactivated concentrate of platelet growth factors is higher than 0.5, preferably higher than 1, preferably higher than 1.5, preferably higher than 2 and preferably higher than 2.5 ng/ml of the concentrate.

In a particular instance the concentration of VEGF in the clottable virally inactivated concentrate of platelet growth factors after SD treatment and oil extraction is higher than 0.5, preferably higher than 0.75, preferably higher than 1 and preferably higher than 1.5 ng/ml of the concentrate.

In a particular instance, the clottable virally inactivated concentrate of platelet growth factors obtained by the method of the invention is suitable for use in conditions requesting a low lipid content.

As used in the present invention, by the expression "in conditions requesting a low lipid content", it is meant that the clottable virally inactivated concentrate of platelet growth factors obtained by the method of the invention is depleted in lipids, i.e. the level of cholesterol, triglycerides, HDL and LDL in the clottable virally inactivated concentrate of platelet growth factors of the invention or obtained by the process of the invention is significantly lower than that of the starting platelet concentrate or than that of a growth factors concentrate obtained by known in the art platelets activation. The extremely low levels in lipids, and in particular in cholesterol, in triglycerides and in LDL of the clottable platelet-derived growth factors concentrate thus render this concentrate suitable for specific therapeutic use, such as, for instance, use in patients presenting elevated risks of cardio-vascular complications, or suffering from high cardiac risk conditions, since the extremely low level of lipids provided to the treated patient strongly decreases the risk for subsequent vascular occlusion resulting from the formation of plaques in the arterial and venous system.

Preferably, the level of cholesterol in the clottable virally inactivated concentrate of platelet growth factors obtained by the process of the invention is lower than 100, more preferably lower than 50 and even more preferably lower than 35 mg/dl of the clottable concentrate of platelet growth factors.

Preferably, the level of triglycerides in the clottable virally inactivated concentrate of platelet growth factors obtained by the process of the invention is lower than 100, more preferably lower than 50 and even more preferably lower than 30 mg/dl of the clottable concentrate of platelet growth factors.

Preferably, the level of HDL in the clottable virally inactivated concentrate of platelet growth factors obtained by the process of the invention is lower than 30, more preferably lower than 15 and even more preferably lower than 10 mg/dl of the clottable concentrate of platelet growth factors.

Preferably, the level of LDL in the clottable virally inactivated concentrate of platelet growth factors obtained by the process of the invention is lower than 80, more preferably lower than 50, more preferably lower than 20, and even more preferably lower than 5 mg/dl of the clottable concentrate of platelet growth factors.

Further, the very low and/or undetectable amounts of lipids in the clottable virally inactivated concentrate of platelet growth factors obtained by the method of the invention has an enhanced stability and is easier to process when large-scale purification of platelet growth factors is implemented. This will be especially useful when anionic and/or cationic chromatography separation processes are carried out.

In a particular instance, the clottable virally inactivated concentrate of platelet growth factors obtained by the method of the invention does not lead to blood cell-related transfusion reactions. By "blood cell-related transfusion reactions", it is meant that this clottable growth factors concentrate is free of intact living cells (such as red cells, platelets and white blood cells, for instance) and therefore allows to avoid a range of well-known transfusion reactions, including immunological (such as alloimmunization) and hemolytic complications, in patients (see, for instance Stroncek et Rebulla, "Platelet transfusions", The Lancet, August 4, 2007; vol. 370: 427-438). Indeed, a recipient who is immunocompetent often exhibits an immune response to the donor blood cell antigens, thus resulting in a variety of clinical consequences depending on the blood cells and specific antigens involved. The antigens most commonly involved are selected in the following categories: (1) HLAs class I, which are shared by platelets and leukocytes, (2) HLAs class II, which are present on some leukocytes, (3) granulocyte-specific antigens, (4) platelet-specific antigens (such as, for instance, human platelet antigen HPA), and (5) Red Blood Cells-specific antigens.

More specifically, as regards the preparation method of the invention, living blood cells (red blood cells, white blood cells, and platelets) are destroyed/lysed by the solvent-detergent treatment, thereby reducing, and preferably preventing, the risk of exposing the patient to foreign antigens and intact blood cells. The method of the invention thus allows to prepare a clottable virally inactivated concentrate of platelet growth factors either from platelets obtained from the patient to be treated itself or from allogeneic platelets.

In a particular instance, the clottable virally inactivated concentrate of platelet growth factors obtained by the method of the invention further comprises at least one protein selected in the group consisting in fibronectin, vitronectin, thrombospondin, and/or in the group consisting in the coagulation factors II (prothrombin), V, VII, VIII, IX, X, XI and von Willebrand factor.

In a particular instance the concentration of fibronectin in the clottable virally inactivated concentrate of platelet growth factors is preferably higher than 0.2 g/L of the concentrate, and preferably about 0.3 g/L of the concentrate.

In a particular instance, the concentration of each of coagulation factors II, IX, X, VII and/or XI in the clottable virally inactivated platelet-derived growth factors concentrate is preferably higher than 0.5 iu/ml of the concentrate, and preferably about 1 iu/ml of the concentrate.

In a particular instance, the concentration of each of coagulation factor VIII and/or von Willebrand factor in the clottable virally inactivated concentrate of platelet growth factors is preferably higher than 0.5, preferably higher than 0.9 iu/ml of the concentrate, and preferably about 1 iu/ml of the concentrate.

In another instance, the clottable virally inactivated concentrate of platelet growth factors obtained by the method of the invention further comprises immunoglobulins such as IgG, IgM, and/or IgA.

In a particular instance the concentration of immunoglobulin IgG in the clottable virally inactivated concentrate of platelet growth factors is preferably higher than 5 g/L of the concentrate, and preferably about 10 g/L of the concentrate.

In another instance the clottable virally inactivated concentrate of platelet growth factors obtained by the method of the invention further comprises at least one growth factor selected in the group consisting in the members of the CCN family, the connective tissue-activating protein-3 (CTAP-3), PF4, the platelet-derived angiogenic factor (PDAF), the endothelial cell growth inhibitor, the early pregnancy factor (EPF), the epithelial growth inhibitor (EGI), the keratinocyte growth factor (KGF), the angiopoietin-like-6 (ANGPTL6), IGFBP-3, estrogen receptor-related proteins, the fibroblast-derived endothelial cell growth factor (f-ECGF), the hepatocyte growth factor (HGF), histamine-releasing factors, human collagenase inhibitor, the platelet microbicidal protein-1 (PMP-1), the thrombin-induced platelet mocrobicidal protein-1 (t-PMP), thrombocidin-1 (TC1) and thrombocidin-2 (TC2).

In another instance the clottable virally inactivated concentrate of platelet growth factors obtained by the method of the invention further comprise at least one protein selected in the group consisting in serotonin, cathepsin, albumin, platelet basic protein-PBP (CXCL7), neutrophil-activating protein-2 and -4 (NAP-2;4), somatostatin (SST), RANTES, CTAP-3, placental protein 14 (PP14), SCUBE1, annexin 11, heat shock protein 27 (HSP27), and heat shock protein 60 (HSP60).

In a particular instance, the concentration of albumin in the clottable virally inactivated concentrate of platelet growth factors is preferably higher than 30 g/L of the concentrate.

In another instance, the clottable virally inactivated concentrate of platelet growth factors obtained by the method of the invention further comprise at least one enzyme selected in the group consisting of collagenase, superoxide dismutase (SOD), heparinase, metalloprotease MMP-1, -2, -9, -13, autocrine ERK (ext.Cell. reg. kinase), autocrine and paracrine protein C (PC), and trace amount enzymes such as aldolase, carboxypeptidases, acid phosphatase, arylsulphatase, β-galactosidase, β-glucoronidase, β-glycerolphosphatase, α/β-glucosidases, α/β-fucosidases, α-mannosidase, and α-arabinosidase.

In another instance, the clottable virally inactivated concentrate of platelet growth factors obtained by the method of the invention further comprise histamine, ADAMTS-13, α1-α2 antitrypsin, α2-antiplasmin, α2-macroglobulin, C1-INH, inducible BMP-2, -6, -7 (TGF-β superfamily), ECM remodelling factors (induced MMP, TNF-α, elastase, ...), autocrine and paracrine lysophosphatidic acid (LPA), HMGB1 (amphiregulin), ATP, ADP, GPT, GDP, Ca2+, Mg2+ and/or Zn2+.

The present application also describes a pharmaceutical product comprising the clottable virally inactivated concentrate of platelet growth factors obtained by the method of the invention for use in medicine.

By "pharmaceutical product", it is meant a platelet gel, a platelet glue, a growth factors-enriched fibrin glue and/or sealant, artificial scaffolds.

The present application also describes the use of the clottable virally inactivated platelet growth factors obtained by the method of the invention in a culture medium which is suitable for in vitro or ex vivo culturing of fibroblasts, chondrocytes, osteoblasts, keratinocytes, stem cells and/or transplants cells. The present application describes a culture medium suitable for in vitro or ex vivo culturing of fibroblasts, chondrocytes, osteoblasts, keratinocytes, stem cells and/or transplants cells, and containing the clottable platelet growth factors obtained by the method of the invention.

An object of the present invention is a method for preparing a clottable virally inactivated concentrate of platelet growth factors according to the claims comprising the steps of contacting non-activated a starting platelet concentrate with a solvent and/or a detergent, incubating said starting platelet concentrate with the solvent and/or detergent for a period of at least 5 minutes to 6 hours, at a pH maintained in a range from about 6.0 to about 9.0, and at a temperature within the range of from 2°C to 50°C, preferably within the range of from 25°C to 45°C, and removing the solvent and/or detergent by oil extraction and/or chromatographic means, said method comprising a further step of inactivating non-enveloped viruses.

In a preferred embodiment, the performed incubation period ranges from 2 to 4 hours, at a physiological pH, e.g. a pH ranging from pH 7.0 to pH 7.5 (when starting platelets are fresh platelets) or at a pH ranging from pH 6.8 to 8.2 (when starting platelets are expired and/or frozen platelets). Advantageously, the incubation temperature is about 31 °C.

Suitable solvents for use in the method of the invention are di- or trialkylphosphates, such as tri-(n-butyl)phosphate, tri-(t-butyl)phosphate, tri-(n-hexyl)phosphate, tri-(2-ethylhexyl)phosphate, tri-(n-decyl)phosphate, di-(n-buty!)phosphate, di-(t-butyl)phosphate, di-(n-hexyl)phosphate, di-(2-ethylhexyl )phosphate, di-(n-decyl)phosphate as well as dialkylphosphates with different alkyl chains. Di or trialkylphosphates having different alkyl chains can be employed, for example ethyl di-(n-butyl)phosphate. An especially preferred trialkylphosphate is tri-(n-butyl)phosphate (TnBP).

Suitable detergents for us in the method of the invention include polyoxyethylene derivatives of fatty acids, partial esters of sorbitol anhydrides, for example the products commercialized under the names "Tween 80" also known as "polysorbate 80", "Tween 20", and non-ionic detergents, such as oxyethylated alkylphenol sold under the name "Triton X-100" or "Triton X-45". Further contemplated detergents are sodium deoxycholate and sulfobetaines, such as

N-dodecyl-N, N-dimethyl-2-ammonio-1-ethane sulphonate. Especially preferred detergents are "Triton X-45", "Triton X-100", "Tween 80" and "Tween 20".

In a particular embodiment of the invention, the starting platelets concentrate is incubated with a solvent and a detergent, like TnBP and Triton X-45.

Advantageously, the final concentration of the solvent or of the detergent, or of each one of the solvent and the detergent, is comprised in a range of from 0.2 to 5%, preferably from 0.2 to 2% in volume with respect to the volume of the platelet concentrate. In a preferred embodiment, the starting platelet concentrate is incubated with 2% TnBP. In another preferred embodiment, the starting platelet concentrate is incubated with 1% TnBP and 1% Triton X-45.

The solvent and/or detergent can be extracted from the biological fluid by oil extraction with pharmaceutical grade oil, and/or by other methods such as column chromatography, such that the remaining concentrate is depleted in solvent and/or detergent.

The pharmaceutical grade oil can be a naturally occurring oil, for example extracted from a plant or an animal, or a synthetic compound of similar structure. Suitable naturally occurring oils include castor oil (also known as ricinus oil), soybean, sunflower oil, cottonseed oil. A preferred synthetic compound is a synthetic triglyceride. Examples of suitable synthetic triglycerides include triolein, tristearin, tripalmitin, trimyristin, and combinations thereof.

The amount of pharmaceutical grade oil is the amount that allows the extraction of at least 80% of lipid soluble process chemicals, the oil being used in an amount of from 2 to 20 weight%, based on the weight of the platelets concentrate lysate, preferably from 5 to 15 weight % and more preferably from 5 to 10 weight %. Oil extraction may be carried out once, preferably twice and more preferably three times, depending in particular on the oil concentration.

The solvent and/or the detergent may also be removed from the platelets concentrate lysate by column chromatography. The column chromatography may also be carried out subsequently to oil extraction or directly after solvent and /or detergent treatment.

Suitable chromatography columns that can be used comprise reversed-phase (hydrophobic interaction) matrices, or protein adsorption matrices such as ion-exchange (anion and cation) matrices and affinity (such as immuno-affinity or immobilized heparin) matrices, or size-exclusion matrices. Preferred reversed phase matrices are a C18 silica packing material, a SDR (Solvent-Detergent removal) hyper D (Pall corporation), polystyrene sorbent (Variant) and Amberlyte (Rohm). tC18 silica packing material is also considered, even if C18 is generally preferred for industrial-scale chromatography. These adsorbents are used to bind the solvent and the detergent while the growth factors elute in the breakthrough fraction. Preferred anion exchange matrices are, depending upon the growth factors to be purified, anion-exchangers gels, such as DEAE-Sephadex A-50, DEAE-Sepharose FF, Q-Sepharose, DEAE-Toyopearl 650M, DEAE-Hyper D. Preferred cation exchange matrices are, depending upon the growth factors to be purified, cation-exchangers gels, such as SP-Sepharose and CM-Sepharose (both being available under a FF grade), SP being preferred. These adsorbents are used to bind the growth factors while the solvent and the detergent elute in the breakthrough fraction.

In a preferred embodiment, an anionic and/or cationic chromatography is performed after the oil extraction .Depending on the selected chromatography, such a solvent and/or detergent removal step can also enhance the growth factors separation. In this respect an anionic chromatography (e.g DEAE) will bind preferably TGF-β1 and EGF whereas a cationic chromatography (e.g. SP) will bind preferably PDGF (AB, AA and BB) and VEGF. Thus the successive use of an anionic and a cationic chromatography enables preparation of fractions enriched in specific GF(s). Such an embodiment is disclosed in Figure 11

In one instance, when an anionic and/or cationic chromatography is performed after the oil extraction and/or after the hydrophobic chromatography, the eluting conditions are determined according to the general knowledge of the skilled person in the art. Preferably, elution is performed with a saline solution comprising from 0.15 M to 2 M NaCl. The saline solution used for eluting can also comprise a mixture of NaCl and a salt (such as phosphate or citrate, for instance) of an alkali or alkaline-earth metal in a range of from 0.15 M to 2 M.

Further, when an anionic chromatography is performed, the pH is selected such as to be higher than the isoelectric point of the Growth Factor(s) of interest. On the contrary, when a cationic chromatography is performed the pH is selected such as to be lower than the isoelectric point of the Growth factor(s) of interest. Preferably, the chromatographic conditions are selected such as to allow to perform the chromatographic separation/purification at a pH which is compatible with the stability and the physiological function of the growth factors of interest, for instance a substantially neutral pH such that denaturation of the growth factors could be avoided.

Alternatively, when an anionic and/or cationic chromatography is performed after the oil extraction and/or after the hydrophobic chromatography, the eluting conditions are set according to the general knowledge of the skilled person in the art. The elution can also be performed with a buffer at a different pH from the one used for the binding of the growth factors onto the resin, depending upon the pHi of the growth factors. The solution used for eluting can be in a pH range of from 4 to 10, preferably from 5 to 9, under conditions maintaining the stability and physiological function of the growth factors.

In one instance, the level of solvent after oil extraction and/or chromatography is of less than 100 ppm, preferably less than 50 ppm, preferably less than 20 ppm, more preferably less than 10 ppm, less than 5 ppm, and even more preferably less than 1 ppm.

In one instance, the level of detergent after oil extraction and/or chromatography is of less than 500 ppm, preferably less than 250 ppm, more preferably less than 100 ppm, more preferably less than 50 ppm, and even more preferably less than 10 ppm.

Following the solvent and/or detergent removal by oil extraction and/or by chromatography, a further step is added to inactivate or remove non-enveloped viruses, such as parvovirus B19 or, possibly, hepatitis A virus (HAV) by nanofiltration and more particularly by nanofiltration using 75-nm, 35-nm, 20-nm, 15-nm, or 10-nm pore size filter membrane.

In one instance, a centrifugation step is performed after oil extraction and/or chromatography for removing cell debris. Advantageously, the centrifugation step is performed at 800 to 20000 x g. for 10 to 30 minutes, and preferably at 10000 x g. for 15 minutes.

In another preferred instance, a clarification step by filtration is performed after oil extraction and/or chromatography for removing cell debris. Advantageously, the filtration step is performed on filters having a gradation from 1 µm to 0.2 µm, thereby also allowing the removal of bacteria.

In a particular embodiment, the platelet concentrate used as starting material in the method of the invention correspond to single or pooled standard platelet concentrates, for example platelet concentrates which were prepared for transfusion. The platelet concentrate may also originate from whole blood buffy-coat isolation. One unit of buffy-coat derived platelet concentrate generally corresponds to 30 to 50 ml. Buffy coat derived platelets may be used as starting material either as a single unit or as a pool of single units, for instance under the form of a therapeutic unit, corresponding to a pool of 4 to 6 single units (as disclosed in the Guide to the preparation, use and quality assurance of blood components - 13th edition (2007), edited by the Council of Europe.

The platelet concentrate may also be obtained by apheresis, cytapheresis or plateletpheresis standard procedures (see for instance the Guide to the preparation, use and quality assurance of blood components, 13th edition (2007), edited by the Council of Europe), and may be obtained using MCS+ (Haemonetics), Trima Accell or COBE Spectra (Gambro) or Amicus (Baxter). An apheresis procedure generally yields a larger volume per donor (corresponding to a 300 ml platelet concentrate), when compared to that obtained through buffy-coat isolation procedures.

In a preferred embodiment, the method of the invention comprises a preliminary step consisting in preparing a starting platelet concentrate before step A) wherein said step is apheresis or buffy-coat isolation from whole blood in one instance point-of-care procedures, such as those using blood cell savers/separators or table top devices are used.

The platelet concentrate used as starting material in the method of the invention may be fresh, i.e. less than 5 or 7 days after collection, expired, i.e. more than 5 or 7 days after collection, or expired and frozen for several weeks at -20°C or colder.

In one instance, the platelet concentrate used as starting material for the method of the invention may further comprise white blood cells and/or red blood cells. The starting platelet concentrate may therefore comprise several differentiated and nonactivated leukocytes such as lymphocytes, neutrophilic granulocytes, and monocytes. Neutrophils and monocytes, in particular, are rich in granules containing myeloperoxidase, which catalyzes the oxidation of chloride to generate hypochlorous acid and other reactive oxygen derivates that act as potent bactericidal oxidants toxic to microorganisms and fungi.

Therefore, when the starting platelet concentrate comprise several differentiated and nonactivated leukocytes, the clottable concentrate of platelet growth factors obtained by the method of the invention further comprises antimicrobial components originating from the leukocytes.

In one instance, leukocytes are eliminated from the platelet concentrate used as starting material, preferably by leukoreduction, such that the proinflammatory effects of the proteases and acid hydrolases contained in white blood cells is avoided. Leukoreduction also allows to reduce the risk of a possible prion contamination.

A normal platelet count in a healthy person is generally comprised between 150000 and 400000 platelets per mm³ of blood, i.e. 150 to 400 x 10⁹ platelets /L. This "normal" platelet count is found in about 95% of healthy people, while the remaining 5% may have a statistically abnormal platelet count (either very low or very high).
When platelets are collected by apheresis, the platelet count is generally higher than 1.2 x 10⁹ platelets per ml for a bag of 250 ml, thereby corresponding to a platelet count of more than about 3 x 10¹¹ platelets per bag (unit).
In a preferred embodiment, the number of platelets in the starting platelet concentrate is 3 to 10 times higher than that usually found in blood.

The present application also describes a method for forming a clot, consisting in mixing the clottable virally inactivated concentrate of platelet growth factors obtained by the method of the invention with thrombin. The present application also describes a method for forming a clot, consisting in mixing the clottable virally inactivated concentrate of platelet growth factors of the invention or obtained by the method of the invention with an activator of the coagulation cascade such as activated FVII, or bovine or human thromboplastin.

Prior to form a clot with the clottable virally inactivated concentrate of platelet growth factors obtained by the method of the invention, an antifibrinolytic agent may be added to the clottable virally inactivated concentrate of platelet growth factors obtained by the method according to the invention to inhibit or slow down the degradation of the fibrin clot by naturally occurring proteolytic enzymes (e.g. plasmin) of the fibrinolytic system.

In one instance, the antifibrinolytic agent is aprotinin, but tranexamic acid or epsilon aminocaproic acid at a concentration of >10 mg/ml might also be used as alternate compounds to aprotinin. Aprotinin is usually provided at a concentration of 3000 KIU or less in the liquid form. The final concentration of aprotinin is generally half of the starting solution after mixing the clottable concentrate of platelet growth factors and thrombin components.

The clottable virally inactivated concentrate of platelet growth factors obtained by the method of the invention may further be mixed with artificial scaffolds, such as those made of collagen, chitosan, ceramics for instance, or with plasma derived fibrin glues or fibrin sealants.

Additional compounds may also be added to the clottable virally inactivated concentrate of platelet growth factors, before mixing with thrombin. Such additional components especially comprise, but are not limited to, chemotherapeutic agents, antibiotics and/or hormones.

The mixing of the clottable virally inactivated concentrate of platelet growth factors obtained by the method of the invention together with thrombin reproduces the last step of the coagulation cascade and leads to the gradual polymerization of fibrinogen for forming clots or insoluble fibrin.

The term "thrombin", as used in the invention, relates to thrombin which may be obtained from any origin, and is preferably directed to thrombin originating from bovine origin, more preferably from human origin if used for therapeutic application in humans. Human CaCl₂ activated plasma or batroxobin (another fibrinogen coagulant protease derived from the venom of the snake *borhrops atrox moogendi),* activated FVII, or human or bovine thromboplastin may also be used as alternate compounds to thrombin.

In one instance, 0.1 to 1 volume of thrombin is added to 1 volume of the clottable concentrate of platelet growth factors of the invention or obtained by the method of the invention.

In another instance, thrombin concentration ranges from 20 IU/ml to 1000 IU/ml. More preferably, the final concentration in thrombin is either about 500 IU/ml to ensure sequential and quick polymerization of fibrinogen into soluble and, then, into stable (insoluble) fibrin, or lower (approximately about 4 to 25 IU/ml when slow polymerization is preferable in specific surgical situations.

In another instance, thrombin is obtained by auto-aCtivation of the fibrinogen-rich growth factor concentrate using 10-50 mM CaCl2 in the presence of surface activators such as metal or glass beads.

The two components, i.e. the clottable virally inactivated concentrate of platelet growth factors obtained by the method of the invention and the thrombin, can be applied sequentially or simultaneously to the repair site by a dual-syringe system, with or without the help of an endoscopic delivery device, by spraying or by absorbable sponges (application methods described in Radosevich et al., "Fibrin sealant: Scientific Rationale, Production Methods, Properties, and Current clinical use", Vox Sanguinis, 1997, 72:133-143 and in Marx G, "Evolution of fibrin glue applicators", Transfus Med Rev, 2003;17(4):287-98 are hereby incorporated by reference).

The present application describes the use of the clottable virally inactivated concentrate of platelet growth factors obtained by the method of the invention in therapeutic application, as well as to form a clot, or for in vitro or ex vivo cells culture. When used for *in vitro* or *ex vivo* cells culture, the clottable virally inactivated concentrate of platelet growth factors obtained by the method of the invention is present in the culture medium in the range of from 1% to 30%, preferably, from 2% to 20% and more preferably from 3% to 10% with respect to the volume of the culture medium.

Main therapeutic applications of the concentrate/thrombin mixture comprise but are not limited to: dental surgery, implantology, orthopaedic and oral surgery, plastic surgery, soft and hard tissue healing and reconstruction, cardiovascular, thoracic, or gastrointestinal surgery, neurosurgery, general surgery/traumatology, ophthalmology, otorhinolaryngology, urology, and surgery in anticoagulated patients or in patients with coagulation defects.

The above and other objects, features and advantages of the present invention will become more apparent from the following description, reference being made to the accompanying figures.

### EXAMPLES

### I-MATERIAL AND METHODS

### I.1-Apheresis platelet collection

Starting platelet concentrates (PCs) were collected from volunteer donors after informed consent using a MCS+ multiple component system (Haemonetics, Braintree, USA). Whole blood was withdrawn through a venous catheter, using an intermittent flow, and anticoagulant (1 ml of Anticoagulant Citrate Dextrose Solution Formula - A per 10 ml of blood). The platelet-rich plasma (PRP) was automatically separated from other blood components by centrifugation and collected into a sterile, single use disposable bag, and the erythrocytes and plasma were returned to the donor. The cycle was repeated until a predefined volume of PRP was obtained (about 300 ml). Starting platelet concentrates were processed as described below within 24 hours after collection.

### 1.2-Blood cell counts

Platelets, while blood cells (WBC), and red blood cells counts were determined using a cell counter (ABC Vet Automatic Blood Counter, ABX Diagnostics, France).

### I.3-Processing of starting platelet concentrates

### a-Study design:

Starting platelets concentrates were processed according to the study design in Figure 1. Briefly, starting platelets concentrates from the same donor (300 ml) were mixed gently and separated into two sub-pools of equal volume (150 ml). Sub-pools 1 were subjected directly to the treatment by S/D treatment without prior activation. Sub-pools 2 were activated in the presence of 23 mM CaCl₂ and beads under conditions that lead to the formation of a platelet gel as described below; the resulting releasates was carefully recovered by pipetting (corresponding to a mean volume of 120 ml due to 30 ml loss in the platelet gel) and then subjected to S/D treatment followed by oil extractions. Samples were taken on the starting platelet concentrates, after S/D treatment of the non-activated platelet sub-pools 1, after activation of the platelet sub-pools 2, and after S/D-treatment of the activated sub-pools 2.

### b-Platelet activation:

Platelet concentrates (sub-pools 2) were activated by adding 1 M CaCl₂ (Sigma; Batch 056k0688) to a final concentration of 23mM, in the presence of beads The mixture was put under mild rotating mixing until the formation of a clot, which typically occurred within 5 to 8 minutes. The mixture was allowed to activate for an additional period of 60 min which, under these experimental conditions, is the duration found to favour optimal platelet-derived growth factors release. The supernatant was separated by decantation from the beads/fibrin clot which had formed. The mean volume of supernatant recovered was about 80% that of the starting platelet concentrate. The supernatant was further processed for S/D treatment.

### c-S/D treatment:

For convenience, the S/D treatment of non-activated sub-pools 1 and activated sub-pools 2 was performed in bags as described in EP 1 685 852 Briefly, a 50%/50% mixture of TnBP (Merck KGaA, Darmstadt, Germany) and Triton X-45 (Sigma, Missouri, USA.) was added to the respective subpools over 15 min, with constant mixing, to achieve a final concentration (v/v) of 1% TnBP and 1% Triton X-45. After complete addition, the S/D-platelet sub-pools mixtures were shaken vigorously for 1 min. The processing bags were then completely immersed into a water-bath to warm the S/D platelet mixture to 25 +/- 0.5 °C and then treated for I hr under constant gentle stirring. At completion of the S/D treatment, soybean oil (Sigma, Missouri, USA) was added at a final concentration of 10% (v/v) to the S/D-platelet sub-pools. The bag was shaken vigorously for 1 min and then put onto a rotating shaker for 15 min. The platelet sub-pools (lower layer) was removed from the oil (upper layer) by decantation (20 min) and transferred by gravity into a second bag to repeat the oil extraction three times. This oil extraction procedure allowed the reduction of TnBP and Triton X-45 to less than 10 and 100 ppm, respectively.

### I.4-Impact of centrifugation and of the type of lysing agents

In order to study the impact of platelet content and type of S/D reagents on platelet-derived growth factors release one experiment was carried out as follows: a platelet concentrate (300 ml) was sub-divided into two sub-pools of 150 ml.

One sub-pool was centrifuged at high speed (10000 x g) in order to pellet the platelets and the supernatant was treated by 1% TnBP-1% Triton X-45. The other sub-pool, that was not centrifuged, was divided into two additional sub-pools of 75 ml, one being subjected to 1% TnBP-1% Triton X- 45 treatment, the other to 2% TnBP. The S/D treatments (incubation and oil extractions) were performed as described above.

### I.5-Bovine thrombin activation experiments

To rule out the hypothesis that CaCl₂ activation did not activate the platelets completely, two platelet concentrates (14 ml) were activated in the presence of 0.23 M CaCl₂ and beads as described above.
After 60 min of mild rotating mixing at room temperature, 10 ml of the platelet concentrate releasate were recovered and 0.5 ml of 1000 International Units (IU)/ml of topical bovine thrombin (Thrombin-JMI, 52604-7102-1, Jones Pharma, Saint-Louis, MO) was added, to obtain a final concentration of about 48 IU/ml. The mixture was put 60 min at room temperature under mild rotating mixing. It was then subjected to a 1% TnBP-1% Triton X-45 treatment and oil extraction. In parallel experiments, two platelet concentrate samples (10 ml) were also subjected to direct activation by 0.5 ml of the same bovine thrombin followed by 60 min mild rotating mixing after gel formation, which occurred within seconds. Samples were taken at the various steps of the experimental process, centrifuged at 10 000 x g and frozen at -80°C until platelet-derived growth factors analysis.

### I.6-Growth factors assays

1-ml samples were taken at each step of the procedures. They were centrifuged at 10000 x g for 15 min (Microfuge® 22R, Beckman Coulter, Fullerton, CA) to pellet the platelets and/or cell debris and obtain cell-free supernatants for platelet-derived growth factors measurements. Parallel experiments were also performed using a centrifugation force of 800 x g for 15 min. Supernatants were then immediately frozen at - 80°C.
Samples were thawed at 37°C and analyzed within 1 h by sensitive and specific commercially available immunoassays. Standards and samples were assayed in duplicate, and mean values were calculated. The results were multiplied by the dilution factor applied to the samples.

### a-PDGF-AB

PDGF-AB was assayed using the Quantikine ELISA kit (#.DHD00B, R&D Systems, Minneapolis, MN). Samples were diluted 100 times in the Calibrator Diluent (RD6-11). The plates were incubated for 2 h, washed, and incubated with enzyme conjugated antibodies to PDGF-AB for an additional 3 h at room temperature. The wells were washed using the Wash Buffer, then the Substrate Solution was added for 20-30 min at room temperature. Wells were protected from light. Stop Solution was added to each well, and the absorptions at 450 nm were determined using a microtiter plate reader. The minimum detectable dose was 1.7 pg/ml.

### b-TGF-β1

TGF-β1 was determined by using the Quantikine ELISA kit (DB100B, R&D Systems). Samples were diluted 100-fold in the Calibrator Diluent (RD5-26). A dilution series of TGF-β1 standards (890207) was prepared in 100-µl volumes in 96-well microtiter plates coated with TGF-β-receptor II. Before analysis of TGF-β1, acid activation and neutralization was performed to activate latent TGF-β1 to the immunoreactive form. For this purpose, 0.5 ml samples were mixed with 0.1 ml of 1N HCl, incubated at room temperature for 10 min, neutralized by an addition of 0.1 ml of 1.2N NaOH/0.5M HEPES (N-[2-hydroxyethyl] piperazine-NO-[2-ethanesulfonic acid]) from Sigma (H- 7523), and centrifuged. The supernatant fraction was then assayed for total TGF-β1 content. Aliquots (50µl) were applied in duplicate to the microtiter plate, which was then covered and incubated for 2 h at room temperature. The wells were then washed, enzyme-conjugated polyclonal antibody to TGF-b1 was added, and incubation continued for 1.5 h at room temperature. Measurements were completed as described above. The detection limit of TGF-β1 was 4.61 pg/ml.

### c-EGF

EGF was assayed using the Quantikine ELISA kit (#.DEG00, R&D Systems, Minneapolis, MN). Samples were diluted 20-fold in Calibrator Diluent (RD6N). 200 µl of Standard, control, or sample was added in the wells. The plates were incubated for 2 h at room temperature. Each well was aspirated and washed by filling with Wash Buffer. EGF conjugate was added to each well, and incubated for 2 h at room temperature, The wells were washed using the Wash Buffer and Substrate Solution (200 µl) was added to each well. The mixture was incubated for 20 minutes at room temperature and protected from light. Stop Solution (50 µl) was added to each well. The optical density of each well was determined within 30 minutes, using a microplate reader (VersaMax™ microplate reader, Molecular Devices, USA) set at 450 nm. The minimum detectable dose was 0.7 pg/ml.

### d-IGF-1

IGF-1 was quantified using a Quantikine ELISA kit (DG100, from R&D Systems). Samples were diluted 100-fold in Calibrator Diluent (RD5-22). The minimum detectable dose ranged from 0.007 to 0.056 ng/ml and the mean MDD was 0.026 ng/ml, as reported by the manufacturer. 150 µl of assay diluent (RD1-53) were added to each well, followed by 50 µl of standard (890775). The plates were covered with adhesives strips and incubated for 2 h at 2-8°C. The wells were washed 3 times and then incubated with enzyme-conjugated IGF-1 for 1 h at 2-8°C. Measurements were completed as described above.

### I.7-Statistical analysis

Data for all series of experiments are reported as mean, standard deviation and minimal and maximal values. Statistical comparisons were made with a two-tailed paired Student test. A p value of less than 0.05 was used to assess the significance of the differences in mean PGF concentration between the platelet preparations at different steps of the procedure. Values are presented as non significant (NS; <0.05), < 0.01, or <0.001. Precise p values are indicated when close to 0.05.

### I.8-Preparation and Separation of samples for comparison of PC, S/D-PC and Act-PC

Samples respectively corresponding to the starting platelet concentrate (PC), the starting platelet concentrate treated by solvent/detergent (1% TnBP and 1% Triton X-45) (S/D-PC), and the starting platelet concentrate activated by CaCl₂ (Act-PC) were separated by SDS-PAGE in order to compare protein contents.

20 µg of each sample were mixed with 5µl NuPAGE LDS Sample buffer (4X) (Invitrogen), 2 µl NuPAGE reducing agent (10X) (Invitrogen) and deionized water to obtain a final volume of 20 µl (21µl for the sample corresponding to the activated platelet concentrate). The resulting mixture was then heated for 10 minutes at 70°C and subjected to SDS-PAGE using a 4-12% polyacrylamide gradient gel (NuPAGE Bis-Tris, Invitrogen).

Separation of the proteins was performed at a constant voltage of 200V, during 35 minutes, with an expected current of 150mA/gel. The resulting gels were stained with Coomassie blue R-250.

The protein marker Mark 12 unstained standard (Invitrogen) was used to determine the molecular weight of the proteins contained in the samples. The Mark 12 marker was loaded on a NuPAGE novex 4-12% Bis-Tris gel with MES (Invitrogen), and stained with coomassie blue R-250 after separation. Corresponding results are disclosed in figure 4.

The protein profile of the starting platelet concentrate treated by the solvent/detergent method appears to bear no major difference with that of the untreated starting platelet concentrate, while the protein profile of the activated platelet concentrate differs significantly, as evidenced by the absence of bands in the 40 to 70 kDa regions, which bands correspond respectively to the 63.5, 56 and 47 kDa alpha, beta and gamma subunits of fibrinogen.

### I.9-Growth factors activity assay

In order to determine whether the growth factors resulting from the S/D treatment of platelets kept their activity (1% TnBP - 1% Triton X-45), *in vitro* cell culture studies were performed using the human osteoblast-like MG-63 cell line. The cellular response of MG-63 cells subjected to growth factors concentrates resulting either from S/D-PC or from Act-PC was thus assessed by investigating cell morphology and viability.

10⁵ to 10⁶ cells were cultured in 35-mm Petri-dishes using 90% Minimum essential medium Eagle (MEM) with 2 mM-glutamine, Eagle's BSS (Balanced Salt Solution) adjusted to 1.5 g/L sodium bicarbonate, 0.1 mM non-essential aminoacids, 1.0 mM sodium pyruvate, 10% heat-inactivated fetal bovine serum, and optionally with 5% of the S/D-PC or of the Act-PC growth factors concentrates. After incubation in a humidified atmosphere comprising 5% CO₂ and 95% air at 37°C, the cells were washed with phosphate-buffered saline (PBS, Gibco,UK), then detached with a trypsin-EDTA solution (0.25% trypsin) at 37°C for 5 min, centrifuged and suspended for further cell test.

3-(4,5-dimethylthiazol-2-yl)-2,5-diphenyltetrazolium bromide (MTT) assay was used for detecting living/dead cells (viability assay). Electron microscopic observations were also performed for investigating cells morphology.

3-(4,5-dimethylthiazol-2-yl)-5-(3-carboxymethoxyphenyl)-2-(4-sulfophenyl)-2H-tetrazolium (MTS) assay was used for monitoring cell proliferation. Briefly, 500 µl of MTS (Celliter 96 Promega Corp., USA) was added to each sample. After incubation at 37°C for 60 min., absorbance at 490 nm was measured using a microplate reader.

### I.10-Virus inactivation assay

Viral validation studies of the solvent and/or detergent treatment used for the lysis of the starting platelet concentrate were conducted by performing down-scaling experiment following international Guidelines such as those from EMEA and the recommendations from the WHO. 50 ml of a starting platelet concentrate were spiked independently with stock suspension of a relevant virus (HIV; human immunodeficiency virus) and three model viruses, bovine viral diarrhoea virus (BVDV), which is a model for hepatitis C virus, pseudorabies virus (PRV), which is sometimes used as a model for HBV in the absence of another convenient *in vitro* model virus, and vesicular Stomatitis virus (VSV). High titer stock suspensions of these viruses were introduced into the starting platelet concentrate and a mixture of 1% TnBP and 1% triton X-45 was added. The viral infectivity was evaluated before and at different time points during the Solvent-Detergent treatment to determine the kinetics of viral inactivation. In vitro cell cultures were performed to determine the infectivity and data expressed in TCID₅₀/ml values. The data obtained revealed that the S/D treatment resulted in complete inactivation of the 4 viruses within 5 minutes of treatment (the first time-point evaluated) as no residual viral infectivity was found. Reduction factors obtained were >5.6 log₁₀ for HIV, >6.6 log₁₀ for BVDV, >6.4 log₁₀ for PRV, and >7.0 log₁₀ for VSV. The Solvent and/or Detergent lysis treatment therefore ensures robust viral inactivation of lipid-enveloped viruses potentially present in the starting platelet concentrate lysate.

### I.11-Growth factors composition of expired frozen platelets

Expired (more than 5 days after collection) platelets concentrates were transferred into a freezer at -20°C and were stored for a month. They were then thawed at 35°C in a water bath and experiments identical to that described with fresh platelets were performed. Growth factors content were measured in the various preparations. Corresponding results are disclosed in figures 5 and 6.

### I.12-Clot formation assay

The growth factors concentrate resulting from the starting platelet concentrate treated by S/D was recovered after removal of the solvent and detergent by oil extraction. 5 ml of the resulting growth factors concentrate was introduced in a 5-ml syringe. 5 ml of bovine thrombin at 500 IU/ml was introduced into another 5-ml syringe. Both syringes were positioned on a double-syringe applicator connected to a single nozzle. The two components were forced through it. A platelet gel was formed within 5 seconds.

### II-RESULTS

### II.1-Cell counts

Ten plateletpheresis concentrates were studied from ten different donors. The platelet concentrate resulting from plateletpheresis had a mean of 1064 ± 235.2× 10⁶ platelets/ml (range: 782 - 1358 x 10⁶ platelets/ml), a mean WBC count of 0.1125 ± 0.025 x 10⁶/ml (range: 0·0-1·5) and a mean RBC content of 0.0212 ± 0.025 x 10⁶/ml.

### II.2-Growth factor content

Mean concentration +/- standard deviation (SD), minimum and maximum in PDGF-AB, TGF-β1, EGF, and IGF-1 in the various platelet preparations, and p values are indicated in the Table 1. Individual data points obtained for each series of experiments can be seen in Fig 2.

**Table 1: Mean concentration of growth factors (ng/ml) in the starting platelets (A), after 1%TnBP-1% Triton X-45 treatment (B), and after CaCl₂ activation (C) followed by 1% TnBP-1% Triton X-45 treatment of the releasate (D) - NS: non significant difference**

| | | PDGF-AB (N=10) | TGF-β1 (N=10) | EGF (N=8) | IGF (N=6) |
|---|---|---|---|---|---|
| **A** | m | **13.8** | **16.6** | **<0.0007** | **83.4** |
| | SD | 14.3 | 14.3 | - | 32.8 |
| | Min | 2.2 | 1.1 | - | 60.1 |
| | Max | 49.5 | 36.0 | - | 162.7 |
| **B** | m | **184.4** | **192.2** | **2.2** | **88.4** |
| | SD | 80.2 | 37.4 | 1.6 | 33.5 |
| | Min | 107.7 | 140.4 | 0.7 | 68.8 |
| | Max | 392.6 | 272.6 | 5.0 | 170.1 |
| **C** | m | **84.6** | **63.8** | **0.9** | **117.2** |
| | SD | 35.5 | 14.1 | 0.6 | 34.9 |
| | Min | 52.8 | 48.6 | 0.2 | 82.3 |
| | Max | 209.5 | 88.5 | 1.6 | 195.3 |
| **D** | m | **88.3** | **68.6** | **1.40** | **112.4** |
| | SD | 45.9 | 27.2 | 1.0 | 39.7 |
| | Min | 52.0 | 38.3 | 0.5 | 73.0 |
| | Max | 209.5 | 132.1 | 3.0 | 202.1 |
| **p value** | B vs A | <0.001 | <0.001 | <0.05 | 0.025 |
| | C vs A | <0.001 | <0.001 | <0.05 | <0.001 |
| | B vs C | <0.001 | <0.001 | 0.044 | <0.001 |
| | B vs D | <0.001 | <0.001 | 0.087 | <0.01 |
| | C vs D | NS | NS | NS | NS |

The mean PDGF-AB content in the starting platelet concentrate was 13.8 +/- 14.3 ng/ml (N=10). After direct S/D treatment by TnBP-Triton X-45, the content increased significantly (p <0.001) to 184.4 +/- 80.2 ng/ml, corresponding approximately to a 13-fold increase when compared to the starting platelet concentrate. When the starting platelet concentrates were first activated by CaCl₂, the content was also significantly increased (84.6 +/- 35.5; p < 0.001) but less (about 6 fold increase), and it remained essentially unchanged (88.3 +/- 45.9; NS) during subsequent S/D treatment. The content of PGDF-AB was significantly higher in non-activated S/D-treated platelets than in activated and in activated/S/D-treated platelets (p < 0.001).

Similar data were found for TGF-ß1. The mean TGF-ß1 content in the starting platelet concentrate was 16.6 +/- 14.3 ng/ml (N=10). After direct S/D treatment, the TGF-β1 content increased almost 12-fold compared to the starting PC to 192.2 +/- 37.4ng/ml (p <0.001). When the starting platelet concentrates were first activated by CaCl₂, the content increased about 4-fold (63.8 +/- 14.1 ng/ml) (p < 0.001) and remained non significantly different during the subsequent S/D treatment (68.6 +/- 27.2 ng/ml). Again, the mean content of TGF-ß1 was significantly higher (p < 0.001) in S/D-treated platelets than in activated and activated/S/D-treated platelets.

EGF was undetectable (< 0.7 pg/ml) in the starting platelet concentrate but became detectable after S/D treatment with a mean EGF content (2.2 +/- 1.6 ng/ml; N=6) which was significantly higher (p<0.05) than that obtained after CaCl₂ activation(0.9 +/- 0.6 ng/ml) (p < 0.05), or after CaCl₂ activation followed by subsequent S/D treatment (1.4 +/- 1.0 ng/ml).

The mean IGF-1 content was 83.4 +/- 32.8 ng/ml in the starting platelet concentrate (N=8), Contrary to the other platelet-derived growth factors, it did not seem to increase significantly after the S/D treatment (88.4 +/- 33.5; p = 0.025). The mean content was even slightly (p < 0.001) higher in the platelet preparations after CaCl₂ activation (117.2 +/- 34.9 ng/ml) and remained stable after the subsequent S/D treatment (112.4 +/- 39.7 ng/ml).

The total amount of PDGF-AB, TGF-ß1, EGF, and IGF-1 recovered from 150 ml of platelet concentrate was 28213, 29406, 336, and 13525 ng after direct S/D treatment (153 ml) and 10152, 7156, 108, and 14064 ng after CaCl₂ activation, respectively, taking into account the 20% mean volume loss due to platelet gel formation (120 ml). This confirms the higher efficiency of the S/D treatment to release PDGF-AB, TGF-ß1, and EGF when compared to CaCl₂ activation.

Table 2 compares the content in platelet-derived growth factors in a starting fresh platelet concentrate (A), after centrifugation (to pellet and remove the platelets) followed by TnBP-Triton X-45 treatment of the supernatant (B), and after S/D treatment of the starting platelet concentrate by 1%TnBP-1%Triton X-45 (C) or 2% TnBP (D). Content of PDGF-AB, TGF-ß1, EGF, and IGF-1 in the unprocessed platelet concentrate (A) was consistent with previous data (Table 1). When the platelet-free supernatant (B) was subjected to TnBP-Triton X-45 (B), the content in PDGF-AB, TGF-ß1, or EGF remained low or undetectable (3.9; 1.1; p < 0.001), whereas the content on IGF-1 was high but similar to that of the starting platelet concentrate (75.4 versus 72.2 ng/ml). Therefore, the release of PDGF-AB, TGF-ß1 and EGF during the S/D treatment was due to the presence of platelets. The increase in platelet-derived growth factors content in the platelet lysate treated with 1% TnBP-1% Triton X-45 or 2% TnBP was similar. Further, platelet concentrates subjected to a treatment with either 1% triton X-45 or 1% triton X-100, the detergent being subsequently removed by tC18 adsorption in batch (SP1T45tC18 and SP1T100tC18) without oil extraction, showed that the release of growth factors PDGF-AB, TGF-β1, IGF and EGF felt within the range observed after treatment with 2% TnBP alone or with 1% TnBP and 1% Triton X-45, thus indicating that the extent of platelet lysis appears essentially to be the same using either this combination of solvent and detergent or the solvent or the detergent alone.

Finally, data (not shown) indicated that the centrifugation of the samples at 10000 x g or at 800 x g did not have an impact on the amount of platelet-derived growth factors measured in the assays.

**Table 2: Concentration in various growth factors (ng/ml) of the starting platelet concentrate (A), after TnBP-Triton X-45 treatment of the centrifuged platelet-free supernatant (B), after TnBP-Triton X-45 (C) or 2% TnBP (D) treatments of the starting platelet concentrate.**

| | **PDGF-AB** | **TGF-ß1** | **EGF** | **IGF-1** |
|---|---|---|---|---|
| **A** | 2.9 | 1.1 | <0.0007 | 72.2 |
| **B** | 3.9 | 1.1 | <0.0007 | 75.4 |
| **C** | 128.7 | 207.9 | 0.8 | 79.9 |
| **D** | 133.7 | 185.6 | 1.2 | 89.8 |

Further, the concentration of fibrinogen, fibronectine, albumin, Imunoglobulin IgG, as well as of coagulation factors II, VII, VIII, IX, X, Xl, XIII and of von Willebrand factor were measured in the growth factors concentrates resulting both from the S/D treatment of the starting platelet concentrate and in the CaCl₂-actived platelet concentrate. These results are disclosed in Table 3.

**Table 3: Compared concentrations in various proteins of the growth factors concentrates resulting either from the treatment of the starting platelet concentrate with 1% TnBP-1% Triton X-45 according to the invention (S/D-PC) or from the activation of the starting platelet concentrate with CaCl₂ (Act-PC).**

| **Proteins** | **S/D-PC** | **Act-PC** |
|---|---|---|
| Fibrinogen (g/L) | 2.6 | 0.2 |
| Fibronectin (g/L) | 0.33 | 0.12 |
| Factor VIII (iu/ml) | 0.92 | <0.1 |
| Factor II (iu/ml) | 1.2 | <0.1 |
| Factor IX (iu/ml) | 1.02 | <0.1 |
| Factor X (iu/ml) | 1.15 | <0.1 |
| Factor VII (iu/ml) | 1.08 | <0.1 |
| Factor XIII (iu/ml) | 0.87 | <0.1 |
| Factor XI (iu/ml) | 1.35 | <0.1 |
| Von Willebrand factor (iu/ml) | 0.95 | <0.1 |
| Albumin (g/L) | 33.8 | 32.5 |
| IgG (g/L) | 11.5 | 11.2 |

It therefore appears that the activated platelet concentrate is almost completely depleted in fibrinogen and in coagulation factors.

### II.3-Bovine thrombin activation experiments

To rule out the hypothesis that the CaCl₂/beads treatment led only to partial activation of the platelets, therefore potentially explaining the lower PDGF-AB, TGF-ß1, and EGF content in the releasates compared to the S/D lysates, the content of these platelet-derived growth factors were determined (Figure 3) in the releasates of platelet concentrates that were first activated by CaCl₂ and then subjected to further treatment by bovine thrombin (Act-T) and thrombin with further S/D treatment (Act-T-S/D). Figure 3 shows that the PDGFAB (A), TGF-ß1 (B), and EGF (C) content in the starting platelet concentrate (start), after CaCl₂ activation (Act), followed by bovine thrombin activation (Act-T), and S/D treatment (Act-T-SD). It can be seen that additional bovine thrombin activation does not increase the PDGF-AB (A), TGF-β1 I (B), and EGF (C) release when compared to CaCl₂ activation

It is demonstrated for the first time that the release of platelet growth factors, and in particular PDGF-AB, TGF-β1 and EGF is significantly higher when the S/D treatment is performed on platelets than when platelets were activated by calcium and/or thrombin. The S/D treatment presumably induces a lysis of the lipid-rich platelet membranes, leading to the release of the platelet-derived growth factors from the intracellular alpha-granules. The fact that the release of those platelet-derived growth factors is lower when thrombin activation is applied first probably results from the fact that aggregated platelets and part of the released platelet-derived growth factors are entrapped in the fibrin network (known as platelet gel) formed by the thrombin-induced polymerisation of the fibrinogen present in the starting platelet concentrate. Indeed the possibility that CaCl₂ resulted in only partial activation and incomplete clot formation from endogenous thrombin is ruled out by the fact that further addition of about 48 NIH units of bovine thrombin / ml to the activated platelet releasates did not lead to an increased release of platelet-derived growth factors. The complete activation of platelets is also confirmed by the extremely low (or undetectable) concentrations of fibrinogen and of coagulation factors as disclosed above in table 3.

Similarly (not shown) we found that platelet growth factors content in the releasates was similar when performing an activation of the platelet concentrate with bovine thrombin. We also found that the IGF-1 content remains essentially unchanged after S/D treatment, and increased only slightly after calcium/thrombin activation. This result probably reflects the fact that most IGF is present in a free circulating form in plasma and that only a minimal proportion is present in platelets.

Our data show that the lysis of platelet by S/D treatment appears to be a most effective means to release platelet growth factors from intracellular platelet granules into the exudates compared to other methods used such as thrombin activation, freeze-thaw cycles and/or freeze-drying. Earlier studies have found that the mean PDGF levels in whole human blood serum is 17.5 ng/ml and 0.06 ng per 10⁶ platelets. This corresponds to approximately 60 ng/ml in the apheresis platelet concentrate used in our study while we find a mean about three times higher in the S/D-treated platelet concentrate. Our data further show that the enhanced release of PDGF-AB, TGF-ß1 and EGF from platelets is obtained whether the S/D treatment was performed using either the 1%TnBP-1%Triton X-45 combination, 2% TnBP, or 1% triton X-45 or Triton X-100, and that the oil extraction treatment implemented for the removal of the S/D agents does not reduce the content of the 4 platelet growth factors evaluated in the platelet lysates.

### II.4-Compared lipid contents of Growth factors concentrates

The lipid contents of the concentrate of platelet growth factors resulting either from S/D treatment CaCl₂ activation of the starting platelet concentrate were measured and compared with those of starting Platelet concentrate, as disclosed in table 4.

**Table 4: Compared lipid content of growth factors concentrates prepared from the same starting platelet concentrate, by using 0.5% Triton X-100 (Trit-PC), by S/D treatment according to the invention followed by one oil extraction (S/D-PC 1), by S/D treatment according to the invention followed by three oil extractions (S/D-PC 3), or by CaCl₂ activation of platelets.**

| | Cholesterol (mg/dl) | Triglycerides (mg/dl) | HDL (mg/dl) | LDL (mg/dl) |
|---|---|---|---|---|
| Starting PC | 164 | 170 | 33 | 99 |
| Trit-PC | 160 | 162 | 101 | 62 |
| S/D-PC 1 | 32 | 20 | 11 | 0 |
| S/D-PC 3 | 33 | 43 | 2 | 0 |
| Act-PC | 150 | 156 | 31 | 97 |

The lipid content was tested by a Hitachi clinical technology machine. Each tested sample was prepared from the same starting platelet concentrate having a pH value of pH 7.2 and containing 886x10³ platelets/µl, 0.1x10³ white blood cells (WBC)/µl and 0.08x10⁶ red blood cells (RBC)/µl.

As disclosed in table 4, the amount of LDL (Low density lipoproteins), HDL (High density lipoproteins), Triglycerides and Cholesterol is significantly lower in the S/D-PC growth factors concentrate prepared by S/D treatment followed by oil extraction, when compared to Triton-treated platelets or to activated platelets. Further, no significant difference was observed when platelets lysed by S/D treatment were extracted by one or three oil extractions.

The depletion of the growth factors concentrate of the invention in cholesterol, triglycerides and LDL is of great interest for clinical and therapeutic purposes since the role of triglycerides, as well as that of the combination LDL-cholesterol is well known in the development of atherosclerosis and cardiovascular diseases through the formation of plaques into the arterial and venous system, thereby conducing to heart attacks, strokes and peripheral vascular diseases.

The depletion in lipids will also be of great interest for the further anionic and/or cationic chromatographic fractionating techniques that can be carried out on the lipids-depleted concentrate.

### II.5-Growth factors activity

Microscopic analysis demonstrated changes in cells shape, size, and number: numerous spindle-shaped cells were in particular observed when cells were incubated with the platelet-derived growth factors concentrate resulting from S/D-treatment or from CaCl₂ activation of the starting platelet concentrate. Further, cell counting showed a significant increase in the number of MG-63 osteoblasts in a time- and dose-dependent manner when cells were incubated with the SD-PC growth factors concentrate, when compared to Act-PC or to cells which were not incubated with growth factors concentrates. The MTT analysis also showed an increased cellular activity for cells incubated in the presence of the SD-PC growth factors concentrate when compared to Act-PC treated cells. Neither the S/D-PC nor the Act-PC growth factors concentrates exhibited a cytotoxic effect on MG-63 osteoblasts.

This study therefore demonstrates that the growth factors resulting from both S/D-treated platelets or activated-platelets remain active after the selected extraction process. However, our experiments showed a significant increase in cells responses when cells were incubated with the growth factors concentrate obtained by S/D treatment. This improved effect of the concentrate obtained by the process of the invention on MG-63 cells may either result from the increased content in growth factors, and particularly in PDGF, TGF-β1, and EGF (the importance of these factors being known in wound healing and tissue regeneration), and/or from an increased amount of additional biologically active substances which are absent or under-represented in the activated platelets concentrate.

The capacity of the virally inactivated HPGF mixture to stimulate cell growth was also evaluated using human embryonic kidney fibroblast (HEK293A; Invitrogen Corporation, Carlsbad, California, USA) and Statens Seruminstitute rabbit corneal fibroblast (SIRC) (ATCC CCL-60, Bioresource Collection and Research Center, Hsing-chu, Taiwan). The cell lines were maintained at 37°C in a controlled atmosphere containing 5 percent CO₂. Cells were cultured at a density of 2 x 10³ cells per well in flat bottomed 96-well plates (Greiner bio-one, Tokyo, Japan) in a Dulbecco's Modified Eagle's Medium (D-MEM) supplemented with 10% FBS, 0.1 mM non-essential amino acids and 1 mM sodium pyruvate. Cells were allowed to adhere for 18 hours, and then starved for 6 hours in a serum-free medium. After two washes with serum-free medium, cells were cultured for up to 5 days in the D-MEM medium supplemented with 10% FBS (Gibco, Invitrogen), activated platelet releasate, or HPGF (prepared from PC with SD-treatment, oil extraction and hydrophobic chromatography). Cell cultures were also performed using various concentrations of the HPGF fraction to detect any possible sign of cell toxicity and to evaluate the optimal dose to promote cell growth in the absence of FBS. Cell growth was determined using the MTS tetrazolium compound from Promega Corporation (Madison, Wisconsin, USA) following manufacturer's instructions.

The results of the MTS assays obtained with HEK293A cells are shown in Fig 16. Good viability was found when the cells were cultured in D-MEM supplemented by 10% (v/v) FBS. There was no cell growth in the absence of FBS (B). Cell viability was also good when cultured in the presence of 10% (v/v) of activated PC and HPGF mixtures after tC18, C18, or SDR, respectively, suggesting both cell growth stimulation as well as absence of toxicity. Fig 16 shows that MTS improved in a dose-dependent manner when increasing concentration of HPGF from 3 to 20%. Even at a concentration of 20%, the HPGF mixture did not exhibit toxicity to the cells. The MTS data obtained with SIRC cells are in Fig 16. HPGF was able to stimulate SIRC proliferation. Interestingly, all HPGF concentration tested (up to 20%) stimulated cell growth, but optimal proliferation, comparable to that obtained with 10% FBS, was found when using 0.1 to 0.5% HPGF.

HPGF supported cell growth and maintained cell viability. HPGF at 10% (v/v) in D-MEM medium stimulated the proliferation of human HEK293A fibroblast cell line. Cell growth was similar to that observed using 10% activated PC releasate or 10% FBS. This indicated that (a) the physiological activity of the HPGF was not altered during the viral inactivation and S/D removal process and that the final preparation did not exhibit cell toxicity. MTS values increased with higher concentrations of HPGF from 3% to 20% (v/v). HPGF also supported the growth of the rabbit SIRC cell line in a manner comparable to FBS. Interestingly, the most effective HPGF concentrations at day 5 were as low as 0.3 to 0.5%. The normal cell growth and morphology evidenced that the HPGF did not induce toxicity. Further, this assay confirms that enriching growth medium with platelet lysates supports cell growth and maintains cell viabilities, allows in vitro expansion of human MSCs, enhances their osteogenic, chondrogenic and adipogenic differentiation potential, decreases the time required to reach confluence, and increases colony-forming unit-fibroblast size, as compared to FBS.

These results also confirm the important potential of the growth factors concentrate of the invention or obtained by the method of the invention in therapeutic treatments, as well as for preparing improved cells culture media. Indeed, the Growth factor concentrate of the invention maintains the cell growth stimulation activity of platelet releasates without generating toxicity. Therefore it can be considered as a candidate to substitute for FBS and activated platelet releasates in cell therapy and regenerative medicine.

### II.6 - Preparation of a GF concentrate depleted in fibrinogen

### a. Preparation of the GF mixture

The PC (about 300mL) was processed within 24 hours after collection. It was first subjected to an in-bag solvent/detergent (S/D) treatment using a combination of 1% tri-n-butyl phosphate (TnBP; Merck KGaA, Darmstadt, Germany) and 1% Triton X-45 (Sigma, Missouri, USA) (total = 6 mL). The S/D-PC mixture was shaken vigorously for I min to ensure complete mixing and then immersed into a water-bath at 31°C for at least 1 hr under constant gentle stirring. At completion of the S/D treatment, the S/D-PC mixture was subjected to one extraction with 30 mL (equivalent to 10% v/v) of soybean oil (Sigma, Missouri, USA). After addition of the oil, the bag was shaken vigorously for 1 min and then put onto a rotating shaker for 20 min. The mixture was allowed to decant for 30 min to separate the oil phase from the PC phase. The PC (lower layer; about 280 mL) was recovered by gravity. Upon completion of the oil extraction, the SD-PC was centrifuged at 10,500 rpm ( 10,400 x g) for 15 min at room temperature (20-25°C).

30 mL of the supernatant was processed without dilution by hydrophobic interaction chromatography (HIC) column using an octadecyl (C18; batch WAT020594, dry powder; 125 Ǻ porosity; 55-105µm particle size) packing material from Waters Corporation (Guyancourt, France). The chromatographic material was packed in a column and was washed with 5 volumes of 1M sodium chloride and 20% ethanol, then equilibrated with 5 volumes of 20 mM citrate buffer, pH 7.4. The SD-PC was injected at a ratio of 7 mL per mL of HIC sorbent and a linear flow-rate of 22.6 cm/hr. The C18 effluent (C18-GF) was recovered as soon as the absorption at 280nm increased and till the return of the absorption to the baseline level. Under the experimental setting used, the volume of the breakthrough fraction was 60-80 mL, corresponding to a 2 to 3 dilution factor compared to the volume of SD-PC injected.

Two other HIC sorbents were also evaluated during the course of the study, using the same experimental conditions as described above: a tC18 sorbent (batch WAT036810; dry powder; 125 Ǻ porosity; 36.1-54.2 µm particle size; Waters Corporation) and a SDR HyperD sorbent (batch 20033-0223, 40-100µm particle size; Pall-BioSepra, Cergy Saint Christophe, France). Similar results were obtained.

### b. Removal of fibrinogen by thrombin activation

Fibrinogen (and other coagulation factors) was removed by activation of 10 ml of the growth factor mixture obtained after C18 chromatography by adding either (a) 0.3 ml of 1M CaCl₂ (final concentration of 23mM) and glass beads (to generate endogenous human thrombin) or (b) 1000 IU bovine thrombin to reach a final concentration of about 50 IU/ml. The mixture was put under mild rotating mixing (60 rpm) until the formation of the fibrin clot. The supernatant were recovered, aliquoted, and assessed.

### c. Measurement of fibrinogen

**Table 4**

| **Fraction** | **Fibrinogen (mg/ml)** |
|---|---|
| Growth factor mixture after C18 chromatography | 0.9 |
| After CaCl2 activation | Not detectable |
| After addition of 50IU/ml thrombin | Not detectable |

The data reveals that fibrinogen was completely removed after activation using CaCl2 or using exogenous thrombin

### d. Measurement of growth factors

**Table 5**

| **Fraction** | **PFGF-AB** | **TGF-β1** | **VEGF** |
|---|---|---|---|
| Growth factor mixture after C 18 chromatography | 15.5 | 75.2 | 0.46 |
| After CaCl2 activation | 12.8 | 65.8 | 0.35 |
| After addition of 50IU/ml thrombin | 14.5 | 70.3 | 0.41 |

The data show that growth factors are still present in the supernatant after removal of fibrinogen.

Therefore, the virally-inactivated growth factor mixture deleted from fibrinogen (by the method described above) can potentially be used as a supplement for cell cultures, and in particular for stem cell cultures.

### II.7-Viral inactivation

In addition, there is ample evidence that the conditions of S/D treatment used here efficiently inactivate blood-born enveloped viruses, in particular HIV, HBV, and HCV.

As disclosed above in the material and method, the 1% TnBP-1% Triton X-45 combination at 31°C ensures reduction factors of > 5.6, >6.6, and >6.4 log₁₀ for HIV, BVDV, and PRV within 5 min of treatment, and also rapidly inactivates ≥ 7.0 log₁₀ of VSV and Sindbis model viruses. Non-enveloped viruses, such as HAV and parvovirus B 19, would not be inactivated by the S/D treatment, but they are pathogenic only in a few patients with altered immune functions. The absence of pooling would strongly reduce the statistical risks of contamination of a single-donor allogeneic platelet-derived growth factors preparation, but further nanofiltration may also be implemented to reduce the risk of contamination by non-enveloped viruses. Obviously, platelet-derived growth factors preparations, as described here, would have to be produced in the future under conditions of good manufacturing practices (GMP), for instance by blood establishments (Guidelines on viral inactivation and removal procedures intended to assure the viral safety of human blood plasma products. www.WHO.int. Geneva, 2003:1-72). Such S/D treatment of platelet preparations can offer a number of practical advantages for topical applications. First, the viral safety of allogeneic donations would be improved, allowing wider clinical potential of such products. Second, the higher platelet-derived growth factors titer released by the S/D treatment could possibly improve the cost-effectiveness of procedures using platelet releasates. Third, the viral inactivation method could ease the use of allogeneic platelet-derived growth factors in applications where the use of recombinant platelet-derived growth factors is not approved, or if approved (as for the treatment of some lower-extremity diabetic ulcers) when a combination of natural platelet-derived growth factors may provide beneficial synergistic outcome. Finally, well-characterized virally-inactivated platelet-derived growth factors could also be used for incorporation into fibrin-based or artificial scaffolds for topical applications to hasten the healing process, as a replacement for foetal calf serum or recombinant platelet-derived growth factors for cell engineering studies, or for ex vivo expansion of mesenchymal stem cells and their differentiation into bone cells, or chondrocytes.

### III. Separation of growth factors: Ion exchange chromatographic experiments to remove the SD and separate growth factors fractions

### 1. Preliminary batch adsorption steps to select appropriate chromatographic support and determine binding capacity for various growth factors

### a. Adsorption test of PDGF-AB

The SD-PC was obtained as previously described. 1 oil extraction was done to reduce the level of contamination in TnBP and Triton X-45. 5 ml of SP-Sepharose FF gel, CM-Sepharose FF, DEAE-Sepharose FF, were washed in a column to equilibrate the gels with a 20 mM sodium citrate, 0.05M NaCl buffer at pH 7.5. When the gels were equilibrated (= the pH and conductivity of the effluent are the same as the starting buffer), the columns were unpacked, and the gels were recovered in a beaker. The pH of the SD-PC was adjusted to 7.5 using diluted NaOH. 1g of each gel was poured in separate tubes and the extra buffer was removed. Then various amounts of SD-PC were added to the various gels. Rotate the tubes slowly for 30 min. Let the gel sediment for about 5-10 min. Remove as much as possible of the supernatants and take a sample to measure the content in growth factors.

The starting SD-PC contained 280 ng/ml of PDGF-AB. The data show very clearly that maximum adsorption of PDGF-AB was obtained when using cation-exchange gels SP-Sepharose FF and CM-Sepharose FF at ratio varying from 3 to 10 ml of SD-PC per 1ml of gel. A particularly high adsorption is obtained using SP-Sepharose FF as revealed by the fact that there is a low amount of PDGF-AB in the supernatant after contact with SP-Sepharose FF at a ratio of 10 ml of SD-PC for 1g of gel. By contrast a large quantity of PDGF-AB was found in the supernatants obtained after adsorption of the SD-PC on the DEAE-Sepharose FF (about 250 ng/g).
These data are displayed in Figure 7.

### b. Adsorption tests of VEGF

The SD-PC was obtained as previously described. 1 oil extraction was done to reduce the level of contamination in TnBP and Triton X-45. 5 ml of SP-Sepharose FF gel, CM-Sepharose FF, DEAE-Sepharose FF, were washed in a column to equilibrate the gels with a 20 mM sodium citrate, 0.05M NaCl buffer at pH 7.5. When the gels were equilibrated ( = the pH and conductivity of the effluent are the same as the starting buffer), the columns were unpacked, and the gels were recovered in a beaker. The pH of the SD-PC was adjusted to 7.5 using diluted NaOH. 1g of each gel was poured in separate tubes and the extra buffer was removed. Then various amounts of SD-PC were added to the various gels. Rotate the tubes slowly for 30 min. Let the gel sediment for about 5-10 min. Remove as much as possible of the supernatants and take a sample to measure the content in growth factors.

The figure 8 shows the % of adsorption of VEGF on the various resins based on the ratio of SD-PC to the gel. The data show that there is limited adsorption of VEGF when using the anion-exchanger DEAE-Sepharose FF especially at a ratios of 10 ml of SD-PC per 1g of gel, since essentially no VEGF was adsorbed. By contrast, a higher proportion (about 60% ) of VEGF was adsorbed by the SP-Sepharose FF and the CM-Sepharose FF.

### c. Adsorption tests of TGF-β1

The SD-PC was obtained as previously described. I oil extraction was done to reduce the level of contamination in TnBP and Triton X-45. 5 ml of SP-Sepharose FF gel, CM-Sepharose FF, DEAE-Sepharose FF, were washed in a column to equilibrate the gels with a 20 mM sodium citrate, 0.05M NaCl buffer at pH 7.5. When the gels were equilibrated ( = the pH and conductivity of the effluent are the same as the starting buffer), the columns were unpacked, and the gels were recovered in a beaker. The pH of the SD-PC was adjusted to 7.5 using diluted NaOH. 1g of each gel was poured in separate tubes and the extra buffer was removed. Then various amounts of SD-PC were added to the various gels. Rotate the tubes slowly for 30 min. Let the gel sediment for about 5-10 min. Remove as much as possible of the supernatants and take a sample to measure the content in growth factors.

The Figure 9 shows the % of adsorption of TGF-β1 on the various resins based on the ratio of SD-PC to the gel. The data show that maximum adsorption of TGF-β1 was obtained when using the anion-exchanger DEAE-Sepharose FF at a ratio of 3ml of SD-PC per 1ml of gel, since over 70% of TGF-β1 was adsorbed. By contrast, a small proportion (about 20% or less) of TGF-β1 was adsorbed by the CM-Sepharose FF, the SP-Sepharose FF.

### d. Adsorption tests of EGF

The SD-PC was obtained as previously described. 1 oil extraction was done to reduce the level of contamination in TnBP and Triton X-45. 5 ml of SP-Sepharose FF gel, CM-Sepharose FF, DEAE-Sepharose FF, were washed in a column to equilibrate the gels with a 20 mM sodium citrate, 0.05M NaCl buffer at pH 7.5. When the gels were equilibrated ( = the pH and conductivity of the effluent are the same as the starting buffer), the columns were unpacked, and the gels were recovered in a beaker. The pH of the SD-PC was adjusted to 7.5 using diluted NaOH. 1g of each gel was poured in separate tubes and the extra buffer was removed. Then various amounts of SD-PC were added to the various gels. Rotate the tubes slowly for 30 min. Let the gel sediment for about 5-10 min. Remove as much as possible of the supernatants and take a sample to measure the content in growth factors.

The Figure 10 shows the % of adsorption of EGF on the various resins based on the ratio of SD-PC to the gel. The data show that maximum adsorption of EGF was obtained when using the anion-exchanger DEAE-Sepharose FF at a ratio of 3ml of SD-PC per g of gel, since over 70% of EGF was adsorbed. By contrast, a smaller proportion (15 to 30 %) was adsorbed by the SP-Sepharose FF, and even less (5 to 20%) when using the CM-Sepharose FF.

### e. Conclusion

The cation-exchanger SP-Sepharose FF and CM-Sepharose FF were found to be adapted to the adsorption of PDGF-AB and VEGF, whereas DEAE-Sepharose FF gave satisfactory data for adsorbing TGF-β1 and EGF

### 2. SP-Sepharose FF column chromatography experiments

Further experiments were conducted to confirm the removal of the SD when using ion-exchangers, as follows:

### a. Preparation of the SD-PC

In this experiment, the SD-PC was subjected to one oil extraction. The quantity of SP-Sepharose FF used was 10ml (about 10g) for 100 ml of SP-PC subjected to 1 oil extraction, as determined by the batch adsorption experiments.

### b. Column equilibration

The SP-Sepharose FF was packed in a column (GE Healthcare). After packing the column, the SP-Sepharose FF was equilibrated in a 20 mM citrate, 0.05M NaCl buffer at pH 7.5. This equilibration buffer was run through the column at a linear flow-rate of 50 cm/hr until the pH and the conductivity of the effluent were identical to that of the equilibration buffer. The absorption of the effluent at 280nm was recorded.

### c. SD-PC injection and breakthrough collection

The pH of the SD-PC was adjusted to 7.5 using diluted NaOH and then injected onto the column at a linear flow-rate of 50 cm/hr. The effluent was collected as soon as the A280nm started to increase.

### d. Return to baseline and growth factors elution

When all the SD-PC was injected, the column was washed with the equilibration buffer until the A280nm returns to the initial baseline
The SP-Sepharose FF was then washed by a 20 mM citrate, 1M NaCl buffer at pH 7.5 at 50 cm/hr. The eluate was collected as soon as the A280nm started to increase and until the return to the baseline A280nm level.

### e. Column regeneration and storage

The column was then regenerate by 2 column volumes of 2M sodium chloride, followed by 2 column volumes of 0.5N NaOH.

### f. Results

The Table 6 gives the growth factor content in the column eluate and the table 7 provides with the contents in solvent end detergent.

**Table 6 : Growth factor content (ng/ml)**

| | PDGF-AB | VEGF | PDGF-AA | PDGF-BB |
|---|---|---|---|---|
| SD-PC | 101.8±41.43 | 1.211±0.5113 | 9.342±1.477 | 35.25±12.34 |
| SP-eluate | 238.3±87.09 | 1.18±0.7244 | 3.488±0.755 | 136.7±44.22 |
| SP-effluent | 2.278±1.055 | 0.3624±0.1414 | 1.140±0.5626 | 4.823±0.0 |

The Table 6 shows that the SP-eluate is enriched in PDGF-AB and PDGF-BB and and allows to separate both PDGF-AA and VEGF whereas the TnBP and the Triton X-45 are found in the breakthrough (effluent) fraction (see Table 7). The 1M PDGF-AB/VEGF eluate has non-detectable contamination in these viral inactivating agents.

**Table 7 : Solvent and detergent content**

| | TnBP ppm | Triton X-45 ppm |
|---|---|---|
| SD-PC | 850 | 1500 |
| SP-eluate | < 2 | < 2 |
| SP-effluent | 600 | 1180 |

### 3. DEAE-Sepharose FF column chromatography experiments

### a. Preparation of the SD-PC

The PC (about 300mL) were processed within 24 hours after collection. They were first subjected to an in-bag solvent/detergent (S/D) treatment using a combination of 1% tri-n-butyl phosphate (TnBP; Merck KGaA, Darmstadt, Germany) and 1% Triton X-45 (Sigma, Missouri, USA) (total = 6 mL). The S/D-PC mixture was shaken vigorously for 1 min to ensure complete mixing and then immersed into a water-bath at 31°C for at least 1 hr under constant gentle stirring. At completion of the S/D treatment, the S/D-PC mixture was subjected to one extraction with 30 mL (equivalent to 10% v/v) of soybean oil (Sigma, Missouri, USA). After addition of the oil, the bag was shaken vigorously for 1 min and then put onto a rotating shaker for 20 min. The mixture was allowed to decant for 30 min to separate the oil phase from the PC phase. The PC (lower layer; about 280 mL) was recovered by gravity. Upon completion of the oil extraction, the SD-PC was centrifuged at 10'500 rpm (10'600 x g) for 15 min at room temperature (20-25°C).

### b. Hi-Trap™ DEAE-Sepharose FF

20 mL of the supernatant was processed without dilution by anion exchange chromatography using prepacked ready to use column. The quantity of HiTrap™ DEAE-Sepharose FF used was 5ml for 20 ml SD-PC sample. The column was equilibrated with 5 volumes of 20 mM citrate, 0.05M NaCl buffer at pH 7.5. The SD-PC (20 mL) was injected at a flow-rate of 50 cm/hr followed by the equilibration buffer. The breakthrough fraction (DEAE-BKT) was recovered as soon as the absorption at 280nm increased and till the return of the absorption to the baseline level. The volume of the breakthrough fraction was 49.7 mL. The column was then further washed with 5 volumes of equilibration to completely wash away the SD into the breakthrough fraction, the column was then eluted with 20mM Citrate, 1M NaCl buffer at pH 7.5 and a linear flow rate of 50 cm/hr. This eluate was collected as soon as the absorption at 280nm increased and till the return of the absorption to the baseline level (volume: 10 mL).

Figures 12 and 13 display EGF content expressed in ng/ml and total ng during the preparation process. The corresponding eluate was collected as soon as the absorption at 280nm increased and till the return of the absorption to the baseline level. The volume collected was 17.9 mL. The breakthrough and the eluate were subjected to measurements of the SD agents and the growth factors (see Table 8).

**Table 8 : content in TnBP and Triton X-45**

| | TnBP,ppm | Triton X-45, ppm |
|---|---|---|
| SD-PC after 1 oil | 686 | 2245 |
| DEAE-Breakthrough | 459 | 1580 |
| DEAE Eluate | < 2 | < 2 |

The data show that the DEAE chromatographic step eliminated the S/D agents that were present in the SD-PC after one oil extraction. The S/D agents were collected in the breakthrough and the 1M NaCl eluate was found to contain no detectable amount of these S/D agents.

### c. Content in growth factors VEGF and PDGF-AB

The breakthrough and the eluate were analyzed for their content in growth factors VEGF and PDGF-AB. The figures 14 and 15 show the content in growth factors expressed in total ng.

Data show that PDGF-AB and VEGF were not bound on the DEAE-Sepharose matrix and were present in the breakthrough (in the context of the present application, the terms gel and matrix have the same meaning). By contrast, EGF was adsorbed onto the gel and eluted, free of the SD agents, from the column.

The above-mentioned anionic and/or cationic chromatographic processes can be carried out on the oil-extracted SD-PC, as is examplified above, but can also be applied directly to the SD-PC without oil extraction. It is preferred to carry out an oil extraction, since it reduces the level of contamination in TnBP and Triton X-45, therefore reducing the volume of buffer needed to wash the column, allowing a faster return to the baseline. The oil extraction also reduces the lipid content, which would favour clogging of the chromatographic material, thereby lowering the efficiency of the separation technique. The oil extraction may however be omitted.
The above-mentioned anionic and/or cationic chromatographic processes can therefore be carried out directly after S/D treatment, but preferably after oil extraction or on the breakthrough of the hydrophobic column if one is used after S/D treatment or after oil extraction.

## Claims

1. A method for preparing a clottable virally inactivated concentrate of platelet growth factors containing both fibrinogen and factor XIII, comprising the following steps :
a) contacting a non-activated starting platelet concentrate with a solvent and/or a detergent, wherein said solvent is preferably selected in the group consisting of di- or trialkylphosphates, di or trialkylphosphates with different alkyl chains, and is preferably the tri-n-butylphosphate (TnBP), wherein said detergent is preferably selected in the group consisting of polyoxyethylene derivatives of fatty acids, partial esters of sorbitol anhydrides, non-ionic detergents, sodium deoxycholate and sulfobetaines, and is more preferably Triton® x-45, Triton® X-100 or Tween® 80, and wherein the final concentration of each of said solvent and/or of said detergent preferably ranges from 0.2 to 5 % in volume with respect to the volume of the starting platelets concentrate;
b) incubating said starting platelet concentrate with the solvent and/or detergent for a period of at least 5 minutes to 6 hours, at a pH maintained in a range from about 6.0 to about 9.0, and at a temperature within the range of from 2°C to 50°C, and
c) removing the solvent and/or the detergent by oil extraction and/or chromatographic means, wherein oil extraction is preferably performed with a pharmaceutical grade oil, the oil being used in an amount of from 2 to 20 weight%, or from 5 to 15 weight % or from 5 to 10 weight %, based on the weight of the mixture of the platelet concentrate with the solvent and/or the detergent, and wherein the chromatographic means preferably comprise an hydrophobic (reversed phase) column, preferably a C18 silica packing material or a SDR (Solvent-Detergent removal) hyper D, or an adsorption chromatographic column such as an anionic and/or cationic chromatographic column;
said method comprising a further step of inactivating non-enveloped viruses by nanofiltration.

2. The method according to claim 1, wherein the starting platelet concentrate is contacted either with 2% TnBP only, or with 1% TnBP and 1% Triton X-45, based on the volume of the starting platelet concentrate.

3. The method according to any one of claims 1 to 2, further comprising a further step (c1) wherein the step (c1) comprises at least one anionic and/or cationic chromatography separation, wherein said cationic chromatography is preferably a strong cationic chromatography and/or the anionic chromatography is preferably a weak anionic chromatography.

4. The method according to any one of claims 1 to 3, wherein the step (c) comprises at least one oil extraction, followed by a chromatography on strong cation exchanger, and a chromatography on weak anion exchanger, said strong cation exchanger being preferably a SP-Sepharose and said weak anion exchanger being preferably a DEAE-Sepharose.

5. The method according to any one of claims 1 to 4, comprising a further step d) of concentration by ultrafiltration preferably on membranes with a cut value of 5000 Daltons or less.

6. The method according to any one of claims 1 to 5 wherein the further step of inactivating non-enveloped viruses is performed by nanofiltration using a 10 to 75-nm pore size filter membrane.

7. The method according to any one of claims 1 to 6 further comprising a preliminary step consisting in preparing a starting platelet concentrate before said step a), wherein the starting platelet concentrate is prepared by apheresis or by buffy-coat isolation from whole blood, and wherein said starting platelet concentrate is fresh, expired or expired and frozen.

8. The method according to any one of claims 1 to 7 comprising a further e) step of removing fibrinogen from the mixture of growth factors, preferably by adding CaC12 or thrombin.

9. The method according to any of claims 1 to 8 for the preparation of a clottable virally inactivated concentrate of platelet growth factors suitable for therapeutic and/or cosmetic use.

## Patentansprüche

1. Verfahren zur Herstellung eines gerinnungsfähigen viral inaktivierten Konzentrats von Blutplättchen-Wachstumsfaktoren, welches sowohl Fibrinogen als auch Faktor XIII enthält, das die folgenden Schritte umfasst:
a) in Kontakt bringen eines nicht-aktivierten anfänglichen Blutplättchenkonzentrats mit einem Lösemittel und/oder einem Detergens, wobei das Lösemittel vorzugsweise ausgewählt ist aus der Gruppe bestehend aus Di- oder Trialkylphosphaten, Di- oder Trialkylphosphaten mit unterschiedlichen Alkylketten und vorzugsweise Tri-n-butylphosphat (TnBP) ist, wobei das Detergens vorzugsweise ausgewählt ist aus der Gruppe bestehend aus Polyoxyethylenderivaten von Fettsäuren, partiellen Estern von Sorbitolanhydriden, nicht-ionischen Detergenzien, Natriumdeoxycholat und Sulfobetainen und insbesondere Triton® X-45, Triton® X-100 oder Tween® 80 ist und wobei die Endkonzentration jedes der Lösemittel und/oder der Detergenzien vorzugsweise im Bereich von 0,2 bis 5 Vol.% liegt in Bezug auf das Volumen des anfänglichen Blutplättchenkonzentrats;
b) Inkubieren des anfänglichen Blutplättchenkonzentrats mit dem Lösemittel und/oder Detergens über einen Zeitraum von mindestens 5 Minuten bis 6 Stunden bei einem pH-Wert, der in einem Bereich von etwa 6,0 bis etwa 9,0 aufrecht erhalten wird und bei einer Temperatur im Bereich von 2 °C bis 50 °C, sowie
c) Entfernen des Lösemittels und/oder des Detergens durch Ölextraktion und/oder chromatographische Mittel, wobei die Ölextraktion vorzugsweise mit einem Öl mit pharmazeutischem Grad durchgeführt wird, wobei das Öl in einer Menge von 2 bis 20 Gew.% oder von 5 bis 15 Gew.% oder von 5 bis 10 Gew.% auf Grundlage des Gewichts des Gemisches des Blutplättchenkonzentrats mit dem Lösemittel und/oder dem Detergens verwendet wird und wobei die chromatographischen Mittel vorzugsweise eine hydrophobe (Umkehrphasen)-Säule umfassen, vorzugsweise ein C18 Kieselsäure Packmaterial oder eine SDR (Solvent Detergent Removal) Hyper D- oder eine Adsorptionschromatographiesäule wie etwa eine anionische und/oder kationische Chromatographiesäule;
wobei das Verfahren einen weiteren Schritt des Inaktivierens nicht-umhüllter Viren durch Nanofiltration umfasst.

2. Verfahren nach Anspruch 1, wobei das anfängliche Blutplättchenkonzentrat entweder nur mit 2% TnBP oder mit 1% TnBP und 1% Triton X-45 auf Grundlage des Volumens des anfänglichen Blutplättchenkonzentrats in Kontakt gebracht wird.

3. Verfahren nach einem der Ansprüche 1 bis 2, das ferner den weiteren Schritt (c1) umfasst, wobei der Schritt (c1) mindestens eine anionische und/oder kationische Chromatographietrennung umfasst, wobei die kationische Chromatographie vorzugsweise eine starke kationische Chromatographie ist und/oder die anionische Chromatographie vorzugsweise eine schwache anionische Chromatographie ist.

4. Verfahren nach einem der Ansprüche 1 bis 3, wobei der Schritt (c) mindestens eine Ölextraktion umfasst gefolgt von einer Chromatographie auf einem starken Kationenaustauscher und einer Chromatographie auf einem schwachen Anionenaustauscher, wobei der starke Kationenaustauscher vorzugsweise eine SP-Sepharose ist und der schwache Anionenaustauscher vorzugsweise eine DEAE-Sepharose ist.

5. Verfahren nach einem der Ansprüche 1 bis 4, das einen weiteren Schritt d) des Konzentrierens durch Ultrafiltration umfasst, vorzugsweise auf Membranen mit einem Trennwert von 5000 Dalton oder weniger.

6. Verfahren nach einem der Ansprüche 1 bis 5, wobei der weitere Schritt des Inaktivierens nicht-umhüllter Viren durch Nanofiltration unter Verwendung einer Filtermembran von 10 bis 75 nm Porengröße durchgeführt wird.

7. Verfahren nach einem der Ansprüche 1 bis 6, das ferner einen vorläufigen Schritt umfasst, der in der Herstellung eines anfänglichen Blutplättchenkonzentrats vor Schritt a) besteht, wobei das anfängliche Blutplättchenkonzentrat durch Apherese oder durch Buffy-Coat Isolierung aus Gesamtblut hergestellt wird und wobei das anfängliche Blutplättchenkonzentrat frisch, abgelaufen oder abgelaufen und eingefroren ist.

8. Verfahren nach einem der Ansprüche 1 bis 7, das einen weiteren Schritt e) des Entfernens von Fibrinogen aus dem Gemisch von Wachstumsfaktoren umfasst, vorzugsweise durch Zugabe von CaCl₂ oder Thrombin.

9. Verfahren nach einem der Ansprüche 1 bis 8 zur Herstellung eines gerinnungsfähigen viral inaktivierten Konzentrats von Blutplättchen-Wachstumsfaktoren, das zur therapeutischen und/oder kosmetischen Verwendung geeignet ist.

## Revendications

1. Procédé de préparation d'un concentré coagulable viralement inactivé de facteurs de croissance plaquettaires contenant à la fois du fibrinogène et du facteur XIII, comprenant les étapes suivantes :
a) la mise en contact d'un concentré plaquettaire de départ non activé avec un solvant et/ou un détergent, où ledit solvant est choisi de préférence dans le groupe constitué de phosphates de di- ou trialkyle, de phosphates de di- ou trialkyle avec des chaînes alkyle différentes, et est de préférence le phosphate de tri-n-butyle (TnBP), où ledit détergent est choisi de préférence dans le groupe constitué de dérivés de polyoxyéthylène d'acides gras, d'esters partiels d'anhydrides de sorbitol, de détergents non ioniques, de désoxycholate de sodium et de sulfobétaïnes, et est de manière davantage préférée le Triton^{®} X-45, le Triton^{®} X-100 ou le Tween^{®} 80, et où la concentration finale de chacun dudit solvant et/ou dudit détergent se situe de préférence dans la plage de 0,2 à 5 % en volume par rapport au volume du concentré plaquettaire de départ ;
b) l'incubation dudit concentré plaquettaire de départ avec le solvant et/ou le détergent pendant une période d'au moins 5 minutes à 6 heures, à un pH maintenu dans une plage d'environ 6,0 à environ 9,0, et à une température au sein de la plage de 2 °C à 50 °C, et
c) l'élimination du solvant et/ou du détergent par une extraction par une huile et/ou un moyen chromatographique, où l'extraction par une huile est réalisée de préférence avec une huile de qualité pharmaceutique, l'huile étant utilisée dans une quantité de 2 à 20 % en poids, ou de 5 à 15 % en poids ou de 5 à 10 % en poids, en se basant sur le poids du mélange du concentré plaquettaire avec le solvant et/ou le détergent, et où le moyen chromatographique comprend de préférence une colonne hydrophobe (en phase inverse), de préférence un matériau de remplissage de silice C18 ou une SDR (élimination de solvant-détergent) hyper D, ou une colonne chromatographique d'adsorption comme une colonne chromatographique anionique et/ou cationique ;
ledit procédé comprenant une autre étape d'inactivation de virus sans enveloppe par nanofiltration.

2. Procédé selon la revendication 1, dans lequel le concentré plaquettaire de départ est mis en contact soit avec 2 % de TnBP uniquement soit avec 1 % de TnBP et 1 % de Triton X-45, en se basant sur le volume du concentré plaquettaire de départ.

3. Procédé selon l'une quelconque des revendications 1 ou 2, comprenant en outre une autre étape (c1) où l'étape (c1) comprend au moins une séparation par chromatographie anionique et/ou cationique, où ladite chromatographie cationique est de préférence une chromatographie cationique forte et/ou la chromatographie anionique est de préférence une chromatographie anionique faible.

4. Procédé selon l'une quelconque des revendications 1 à 3, dans lequel l'étape (c) comprend au moins une extraction par une huile, suivie d'une chromatographie sur un échangeur cationique fort, et d'une chromatographie sur un échangeur anionique faible, ledit échangeur cationique fort étant de préférence un SP-Sepharose et ledit échangeur anionique faible étant de préférence un DEAE-Sepharose.

5. Procédé selon l'une quelconque des revendications 1 à 4, comprenant une autre étape d) de concentration par ultrafiltration, de préférence sur des membranes avec une valeur seuil de 5000 daltons ou inférieure.

6. Procédé selon l'une quelconque des revendications 1 à 5, dans lequel l'autre étape d'inactivation de virus sans enveloppe est réalisée par nanofiltration en utilisant une membrane de filtre à taille de pore de 10 à 75 nm.

7. Procédé selon l'une quelconque des revendications 1 à 6, comprenant en outre une étape préliminaire consistant à préparer un concentré plaquettaire de départ avant ladite étape a), où le concentré plaquettaire de départ est préparé par aphérèse ou par isolement de la couche leucocytaire du sang total, et où ledit concentré plaquettaire de départ est frais, périmé ou périmé et congelé.

8. Procédé selon l'une quelconque des revendications 1 à 7, comprenant une autre étape e) d'élimination du fibrinogène à partir du mélange des facteurs de croissance, de préférence par addition de CaCl₂ ou de thrombine.

9. Procédé selon l'une quelconque des revendications 1 à 8, pour la préparation d'un concentré coagulable viralement inactivé de facteurs de croissance plaquettaires approprié pour un usage thérapeutique et/ou cosmétique.
